# EUROPEAN PATENT SPECIFICATION

(11) **EP 2 619 578 B1**
(45) Date of publication and mention of the grant of the patent: **14.12.2016**
(21) Application number: 11827086.7
(22) Date of filing: 20.09.2011
(51) Int. Cl.: G01N 33/53, C40B 30/04

(54) **METHOD OF ANALYZING BINDING INTERACTIONS**
VERFAHREN ZUR ANALYSE VON BINDUNGSINTERAKTIONEN
PROCÉDÉ D'ANALYSE D'INTERACTIONS DE LIAISON

(30) Priority: 22.07.2011 US 201161510876 P; 13.01.2011 US 201161432529 P; 05.04.2011 US 201161472164 P; 24.09.2010 US 386452 P
(43) Date of publication of application: 31.07.2013
(60) Divisional application: 16195171.0
(73) Proprietor: Full Spectrum Genetics, Inc., Belmont, CA 94002 (US)
(72) Inventor: DUBRIDGE, Robert, B., Belmont, CA 94002 (US)
(74) Representative: Herzog, Fiesser & Partner Patentanwälte PartG mbB
(86) International application number: PCT/US2011/001618
(87) International publication number: WO 2012/039756

(56) References cited:
- US-A1- 2002 042 081
- US-A1- 2008 268 420
- US-A1- 2010 093 563
- US-B2- 7 732 195
- FINLAY W J ET AL: "Affinity Maturation of a Humanized Rat Antibody for Anti-RAGE Therapy: Comprehensive Mutagenesis Reveals a High Level of Mutational Plasticity Both Inside and Outside the Complementarity-Determining Regions", JOURNAL OF MOLECULAR BIOLOGY, ACADEMIC PRESS, UNITED KINGDOM, vol. 388, no. 3, 8 May 2009 (2009-05-08), pages 541-558, XP026046499, ISSN: 0022-2836, DOI: 10.1016/J.JMB.2009.03.019 [retrieved on 2009-03-13]

## Description

### BACKGROUND

Great effort has been directed to understanding and manipulating protein-protein and protein-ligand binding reactions because of the central role such reactions play in living systems and in drug development. In particular, a wide range of techniques have been developed to identify or improve the binding reactions of antibodies for therapeutic, diagnostic, analytical and chromatographic applications, e.g. Nieri et al, Current Clinical Medicine, 16: 753-779 (2009); Rajpal et al, Proc. Natl. Acad. Sci., 102: 8466-8471 (2005); Dubel et al, Trends Biotechnology, 28: 333-339 (2010); and the like. A common approach has been to construct comprehensive display libraries that contain a maximum of sequence diversity (e.g. as high as 10¹⁰-10¹¹ independent clones) to increase the chance of identifying antibodies of the highest possible specificity and affinity for a particular antigenic determinant, e.g. Winter et al, Annu. Rev. Immunol., 12: 433-455 (1994); Mondon et al, Frontiers in Bioscience, 13: 1117-1129 (2008); Sidhu et al, Nature Chemical Biology, 2: 682-688 (2006); Carmen et al, Briefings in Functional Genomics and Proteomics, 1: 189-203 (2002); Kretzschmar et al, Current Opinion in Biotechnology, 13: 598-602 (2002); and the like. A typical procedure is to carry out a series of physical selections, for example, using a phage-display library, where candidate phages are repeatedly bound to antigen, washed, eluted, and amplified for another round of selection. After multiple such rounds, a subset of phage is isolated and sequenced to identify candidate antibodies with desired properties, such as high affinity to the antigen, Krebs et al, J. Immunol. Methods, 254: 67-84 (2001); Turunen et al, J. Biomol. Screen., 14: 282-293 (2009). Although such procedures are a huge advance over previous methods requiring generation and screening of hybridomas, they still require significant labor and typically provide only limited information about many other properties of interest, such as molecular information about non-binders, specificity, cross-reactivity, immunogenicity, stability, manufacturability, or comparative measures of performance with respect to wild-type molecules, or other molecular standards or references. Likewise, in studies of general protein-protein or protein-ligand interactions, such information is lacking in current approaches.

The strength of the binding interaction between a protein and its ligand is characterized by its binding affinity, a function of the ratio under equilibrium conditions of ligand bound to protein and the product of free ligand and free protein. One way to measure a protein's binding affinity for its ligand is to mix a known quantity of the protein with decreasing concentrations of the ligand, allow these reactions to reach equilibrium and measure the concentrations of bound versus free protein in each reaction. These measurements can then be used to rank the binding affinities of multiple proteins or protein variants that all bind the same ligand. The protein that has the highest percent binding at any given concentration of ligand will have the highest binding affinity, e.g. Alberts et al, Molecular Biology of the Cell, 4th Edition (Garland Science, New York, 2002). This type of reaction has been run serially on numerous different proteins to compare their binding affinities to a given ligand. A good example of this technique is the radioligand binding assay, e.g. GraphPad Manual (GraphPad Software, 1996). Unfortunately protein binding sites tend to be large, sometimes comprising dozens of uniquely positioned amino acids that contribute to the affinity of the protein for its ligand. Since each amino acid position can accommodate any of the 20 amino acids, the complete analysis of all combinations of variants in a binding site covering 50 amino acid positions would require the analysis of >10¹⁵ mutants.

In view of the above, applications requiring an understanding of protein binding reactions, such as antibody engineering, would be advanced by the availability of efficient techniques for providing statistically significant information on candidate binding molecules despite the large number of candidates that must be assessed in typical protein-ligand and protein-protein interactions.

### SUMMARY OF THE INVENTION

The present disclosure is directed to methods for analyzing protein-protein and/or protein-ligand binding reactions and for improving such reactions for at least one member of such a binding pair, or for improving other characteristics of at least one member of such a pair, including, but not limited to, stability, specificity, immunogenicity, expressibility, manufacturability, or the like. Aspects and embodiments of the present invention are exemplified in a number of implementations and applications, some of which are summarized below and throughout the specification.

In one aspect the disclosure includes a method of analyzing affinities of a library of binding compounds to one or more ligands, the method comprising the steps of: (a) reacting under binding conditions one or more ligands with a library of binding compounds, each binding compound consisting of or being encoded by a nucleotide sequence; (b) determining the nucleotide sequences of binding compounds forming complexes with the one or more ligands; (c) determining the nucleotide sequences of binding compounds free of ligand; and (d) ordering the nucleotide sequences of the binding compounds in accordance with the affinities of their respective binding compounds for the one or more ligands, wherein the affinities are determined by comparing the number of times a nucleotide sequence is identified among binding compounds forming complexes with the one or more ligands and the number of times the same nucleotide sequence is identified among the binding compounds free of the one or more ligands.

In another aspect, the disclosure includes a method of identifying binding compounds that have similar or equivalent affinities to a ligand as that of a standard, or reference, binding compound, the method comprising the steps of: (a) reacting under binding conditions a ligand with a library of candidate binding compounds and a standard, or reference, binding compound, each candidate binding compound and the standard, or reference, binding compound consisting of or being encoded by a nucleotide sequence; (b) determining the nucleotide sequences of binding compounds forming complexes with the ligand; (c) determining the nucleotide sequences of binding compounds free of ligand; (d) ordering the nucleotide sequences of the binding compounds in accordance with the affinities of their respective binding compounds for the ligand, wherein the affinities are determined by comparing the number of times a nucleotide sequence is identified among binding compounds forming complexes with the ligand and the number of times the same nucleotide sequence is identified among the binding compounds free of the ligand; and (e) identifying among the ordering of nucleotide sequences those nucleotide sequences that are adjacent to (i.e., have affinity values close to) the nucleotide sequence encoding the standard, or reference, binding compound.

In another aspect a method of characterizing affinities of a library of binding compounds for one or more ligands is provided by the steps; (a) reacting under binding conditions one or more ligands with a library of candidate binding compounds, each candidate binding compound comprised of or being encoded by a nucleotide sequence; (b) determining the nucleotide sequences of the candidate binding compounds forming complexes with the one or more ligands; and (c) determining for each binding compound an affinity based on a number of times a nucleotide sequence is identified with a binding compound forming a complex with the one or more ligands and a number of times the same nucleotide sequence is identified with the binding compound free of the one or more ligands. In one embodiment the total number of a binding compound may be determined by sequencing a sample of the library prior to the reaction. In another embodiment the total number of a binding compound is determined by determining the nucleotide sequences of candidate binding compounds free of ligand together with the nucleotide sequences of candidate binding compounds forming complexes with the one or more ligands. In this and other aspects an affinity may be a relative affinity of such binding compound with respect to other binding compounds in the same reaction. Also, in this and other aspects each relative affinity may be based on, or be taken as, a ratio of a number of nucleic acid sequences encoding a binding compound that forms a complex with the one or more ligands and a number of the same nucleic acid sequences encoding the same binding compound free of the one or more ligands in the same reaction, or a ratio of a number of nucleic acid sequences encoding a binding compound that forms a complex with the one or more ligands and a total number of the same nucleic acid sequences encoding the same binding compound in the same reaction.

In its aspects and various embodiments, the invention permits reliable and exhaustive identification of "bio-similar" and "bio-better" binding compounds without the use of large inefficiently accessed libraries or repeated cycles of binding, selection and amplification. That is, the disclosure provides methods for obtaining novel binding compounds having equivalent or enhanced binding characteristics with respect to a reference (or wild type) binding compound (including affinity, specificity, lack of cross-reactivity, or the like), such as a known therapeutic antibody. In accordance with the methods of the disclosure candidate binding compounds having equivalent or superior affinity are readily obtained in a one-step process, after which such compounds may be further analyzed to identify members having improvements of other properties, such as increased stability, increased aggregation resistance, reduced immunogenicity, reduced cross reactivity, better manufacturability, or the like with respect to the reference binding compound.

These above-characterized aspects and embodiments, as well as other aspects and embodiments, of the present invention are exemplified in a number of illustrated implementations and applications, some of which are shown in the figures and characterized in the claims section that follows. However, the above summary is not intended to describe each illustrated embodiment or every implementation of the present invention.

### Brief Descriptions of the Drawings

Fig. 1A is a diagram of a work flow for one embodiment of the invention in which nucleic acids encoding binder and non-binders are sequenced.
Fig. 1B is a diagram of a work flow for another embodiment of the invention in which nucleic acids encoding a library of binding compounds is sequenced and nucleic acids encoding members of the library that bind to targets is sequenced.
Figs. 2A-2B show exemplary frequency distributions of encoding nucleic acids from candidate binding compounds that form complexes target antigen (Fig. 2A) and those that are free (Fig. 2B).
Figs. 2C-2D show orderings of binding compounds with respect to affinity based on the data of Figs. 2A and.
Fig. 2E illustrates the construction for further improvements of a second stage library from a subset of binders from a first stage library.
Fig. 2F illustrates a "heat map" representation of affinity data generated by the method of the invention.
Fig. 3 is a diagram of an immunoglobulin G molecule and its constituent regions.
Figs. 4A-4D illustrate a method of analyzing related CDRs using DNA sequence analyzers with limited read lengths.
Fig. 5 is a genetic map of a phagemid vector with which compound libraries of the invention may be made in one embodiment.

### DETAILED DESCRIPTION OF THE INVENTION

The practice of the present invention may employ, unless otherwise indicated, conventional techniques and descriptions of organic chemistry, molecular biology (including recombinant techniques), cell biology, and biochemistry, which are within the skill of the art. Such conventional techniques include, but are not limited to, preparation of synthetic polynucleotides, monoclonal antibodies, antibody display systems, nucleic acid sequencing and analysis, and the like. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV); PCR Primer: A Laboratory Manual; Phage Display: A Laboratory Manual; and Molecular Cloning: A Laboratory Manual (all from Cold Spring Harbor Laboratory Press); Sidhu, editor, Phage Display in Biotechnology and Drug Discovery (CRC Press, 2005); Lutz and Bornscheuer, Editors, Protein Engineering Handbook (Wiley-VCH, 2009); Hermanson, Bioconjugate Techniques, Second Edition (Academic Press, 2008); and the like.

The disclosure provides a method for obtaining statistically significant information about how structural elements of proteins, e.g. position and identity of amino acid residues in binding domains, relate to functional properties of interest, such as binding affinity, specificity, and the like. Such information is collected by reacting under binding conditions a set of candidate nucleic acid-encoded binding compounds with one or more target molecules, so that complexes form between the one or more target molecules and at least a portion of the candidate binding compounds (referred to herein as "binders"). Sufficient numbers of candidate binders and non-binders are then decoded by high throughput nucleic acid sequencing to give statistically significant data about the binding properties of substantially all the members of the set of candidate binding compounds. In other words, sample sizes are large enough so that the numbers of candidate binders and non-binders decoded and recorded are subject to minimal sampling error. In some embodiments, such sampling error, as measured by coefficient of variation, is less than 10 percent; in some embodiments, it is less than 5 percent; in some embodiments, it is less than 2 percent; and in some embodiments, it is less than 1 percent. As disclosed more fully below, embodiments of particular interest are those in which candidate binding compounds are related to a pre-existing reference binding compound, such as a pre-existing antibody, that binds to a target molecule of interest, such as a therapeutic target. In such embodiments, an object of the invention is to improve one or more characteristics of a reference binding compound by generating library of candidate binding compounds based on minimal changes or mutations of the reference binding compound, which, in turn, permits large scale repetitive sequencing of each library member from a binding reaction to obtain statistically significant binding information on each candidate binding compound of the library. From such information, binding compounds different from the reference binding compound are obtained which have equivalent or higher affinity and which may be subjected to further selection to reduce cross reactivity, reduce immunogenicity, increase solubility, increase stability, or the like.

The statistically significant information is contained in the tabulations of the sequences of nucleic acids encoding the binders and the non-binders. Nucleic acid-encoded binding compounds may be obtained from the various antibody display techniques, aptamers, or the like, such as those described below. In some embodiments, the structural elements that are analyzed are spatially local in the sense that they exert their effects on binding within or near a limited volume of a larger molecule, such as, an enzyme active site, antibody binding site, complementary-determining regions, or the like. In particular, structural elements analyzed in an antibody binding interaction includes CDRs as well as framework regions of antibody variable regions. Alternatively, such information may be collected by first decoding the sequences of members of the total effective library of candidate nucleic acid-encoded binding compounds, (or an adequate sample thereof to ensure nearly complete coverage (e.g. at least 95%, or at least 98%, or at least 99% coverage)), prior to carrying out a binding reaction with the one or more target molecules, or ligands. As used herein, "total effective library" means the total library of nucleic acid-encoded binding compounds, subject to any biases in sequence representation that may arise in the course of expression, e.g. in phage, ribosomes, bacteria, yeast, or the like. A binding reaction is carried out as described above, after which the nucleic acid sequences of only the binders are determined. From this information, a ratio may be formed for each candidate nucleic acid-encoded binding compound that consists of the number of sequence reads among the binders over the number of sequence reads in the total library as a measure of its binding strength or affinity. That is, the larger the value of the ratio of a candidate binding compound, the stronger its affinity for the one or more target molecules and the lower the value of the ratio the lower its affinity. Generally, such ratios and other ratios, such as ratios of binders to nonbinders, provide relative affinities of each of the binding compounds in the reaction with the one or more ligands. Such measures of relative affinities are applicable to all embodiments of the invention.

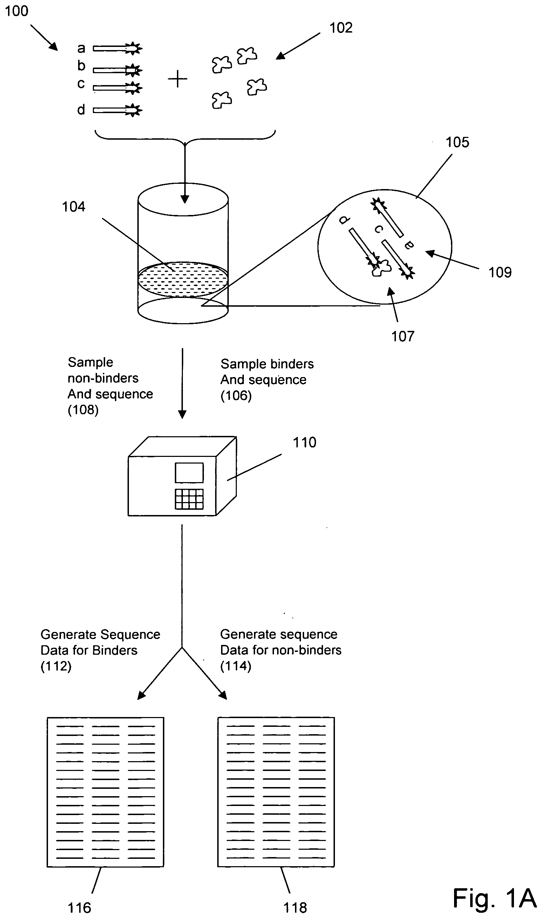

Fig. 1A illustrates a workflow of an exemplary embodiment of the invention. A library (100) of nucleic acid-encoded binding compounds, such as phage displayed antibodies, is combined with antigen (102) in reaction mixture (104) so that a binding equilibrium is established among the compounds. In some embodiments, nucleic acid-encoded binding compounds are present in equimolar concentrations. Components of the reaction mixture, in addition to the binding compounds and antigen, may vary widely. In some embodiments, conventional conditions for antibody-antigen binding are used, e.g. physiological salts at a neutral, or near neutral, pH using a conventional buffer, such as a phosphate buffer. Within the mixture (illustrated by blow-up 105) for each binding compound a fraction will form complexes with antigen (107) and a fraction will remain free (109). In accordance with the invention, a sample of free binding compounds is taken and a sample of antigen-binding compound complexes is taken. For clarity, in some embodiments, such as those using binding compounds displayed on phages, or the like, a sample of free binding compounds means a sample of free phage expressing a binding compound. (Typically free phage will comprise both phage expressing binding compounds that do not bind antigen and phage that simply fail to express any binding compound. The former, that is, free phage expressing binding compound, are readily isolated or separated from phage not expressing binding compound by using conventional techniques, such as separation with anti-constant region antibodies, anti-peptide tag antibodies, e.g. a myc tag or polyhistidine tag (engineered into binding compounds), or like techniques). The two populations are conveniently sampled by using conventional techniques for manipulating proteins or antigens, e.g. Wild, editor, The Immunoassay Handbook, 3rd Edition (Elsevier, 2008). Usually, the antigen is immobilized, or is capable of being immobilized, for example, by direct adsorption to a solid support, such as an assay plate, microtiter well, or the like, or it is indirectly immobilized via a capture antibody that has been immobilized on such a support. For example, antigen may be linked to a solid support, such as magnetic beads, microtiter wells, or the like, or antigen may be labeled with a capture moiety, such as biotin, which permits binding compounds that form complexes to be isolated, e.g. with streptavidin coated magnetic beads, after a binding reaction has reached equilibrium conditions. Nucleic acids encoding the binding compounds forming complexes (i.e. binders) are extracted (106) and sequenced; likewise, nucleic acids encoding the sample of free binding compounds are extracted (108) and sequenced. In order to obtain reliable statistics on the proportion of binders and non-binders the respective samples must be sufficiently large to avoid aberrant results due to sampling error. The appropriate sample size depends at least (i) on the degree of reliability desired in determining the proportions of each binding compound bound or unbound, and (ii) the size of the library of different nucleic acid-encoded binding compounds. Unlike conventional libraries of binding compounds, where maximal diversity is sought, in some embodiments of the present invention, libraries of limited size are employed so that reliable statistics on the binding characteristic of each binding compound can be readily obtained. The size of a library for use with the invention depends on how many residues are varied in the library members, or candidate binding compounds; in other words, the size depends on the number of amino acid positions where amino acids are varied and the number of different amino acids that are substituted in at each such position. For antibodies, varying the amino acids occupying each amino acid position one at a time in a collection of six complementary determining regions (CDRs) leads to about 1600-2200 library members (where "library" here is in reference to the encoded binding compounds, as opposed to the nucleic acids that are translated into amino acids, which of course will be more numerous because of the degeneracy of the genetic codeIn some embodiments of the invention, samples of binders and non-binders for sequencing include many times this number of candidate binding compounds. In some embodiments, sample sizes are in the range of about 5 times or more times the library size. In some embodiments, sample sizes are in the range of from about 5 to 100 times the library size. For a 2000 member library of candidate binding compounds, a sample size of in the range of 10⁴-2x10⁵ may be used, for example. For a library containing about 2.3x10⁴ members (e.g., amino acids of 6 CDRs varied two at a time), a sample size in the range of from 1.1x10⁵ to 2.3x10⁶ may be used. In some embodiments, nucleic acid sequences from such samples are further amplified in the course of sequence analysis. For example, if a Solexa-based sequencer is employed, primer binding sites are attached to sequences from such samples in a PCR which allows bridge PCR for forming clusters on a solid phase surface, which are analyzed by the Solexa-based sequencing chemistry. Preferably, multiple copies (e.g. ≥10 copies) of each sequence from such samples are analyzed to ensure reliable sequence determination. Thus, if a sample size of 10⁴ to 2x10⁵ is used then for Solexa-based sequencing, or equivalent technology, at least 10⁵ to 2x10⁶ clusters are formed, or sequence reads obtained, for data analysis; or if a sample size of 10⁵-10⁶ is used then for Solex-based sequencing, or equivalent technology, at least 10⁶-10⁷ clusters are formed, or sequence reads obtained, for data analysis. In some embodiments, sufficiently large samples are taken so that the measured frequencies have P-values of 0.1 or less, or P-values of 0.05 or less, or P-values of 0.002 or less. In alternative embodiments, nucleic acids encoding scaffold regions may also be used to generate library members either by selective amino acid substitutions, additions, and/or deletions, or by substitution of scaffolds or frameworks from different antibodies, e.g. from different individuals.

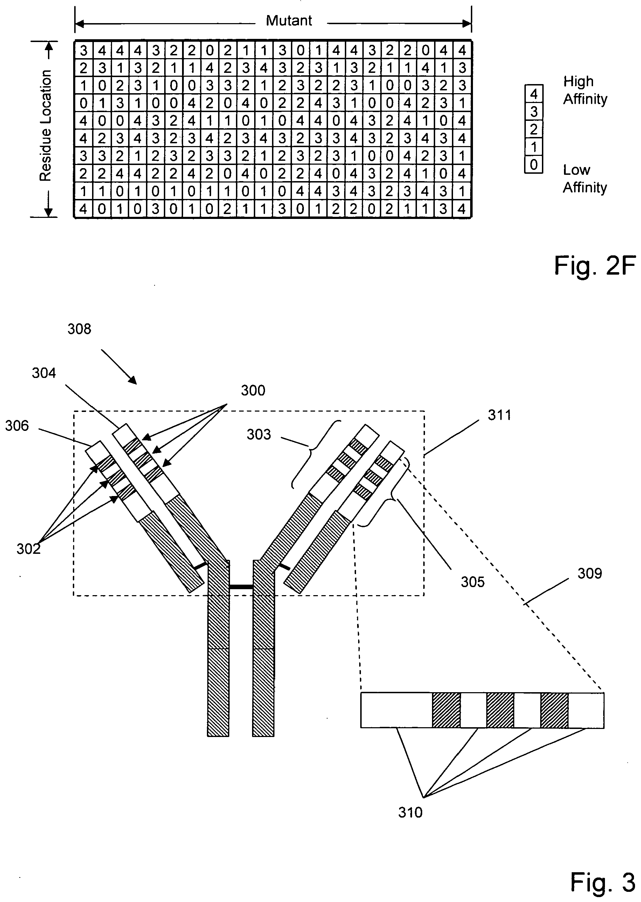

In regard to binding compounds derived from antibodies, Fig. 3 illustrates various functional domains of an IgG antibody, including CDRs (cross-hatched regions)(300) of heavy chain variable region (304) and CDRs (cross-hatched regions) (302) of light chain variable region (306) of antibody (308), which has Fab fragment encompassed by dashed rectangle (311). The other heavy and light chain variable regions of antibody (308) are indicated as (303) and (305), respectively, and "scaffold" or "framework" regions surrounding CDRs of light chain variable region (305) are shown on projection (309) of light chain variable region (305). As described more fully below, in some embodiments, libraries comprise collections of nucleic acids encoding single amino acid mutants of both CDRs and/or framework regions of Fab fragments. The positions of the CDRs and their individual residue in light and heavy chain variable regions are conventionally indicated by various numbering schemes, such as the Kabat, Chothia, Abhinandan numbering schemes, or the like, which permit those of ordinary skill in the art to understand the precise locations of mutants in CDRs and framework regions of antibody-derived binding compounds. Descriptions of such numbering schemes are described in Martin, chapter 2, Kontermann and Dubel (eds.) Antibody Engineering, Vol. 2 (Springer-Verlag, Berlin, 2010).

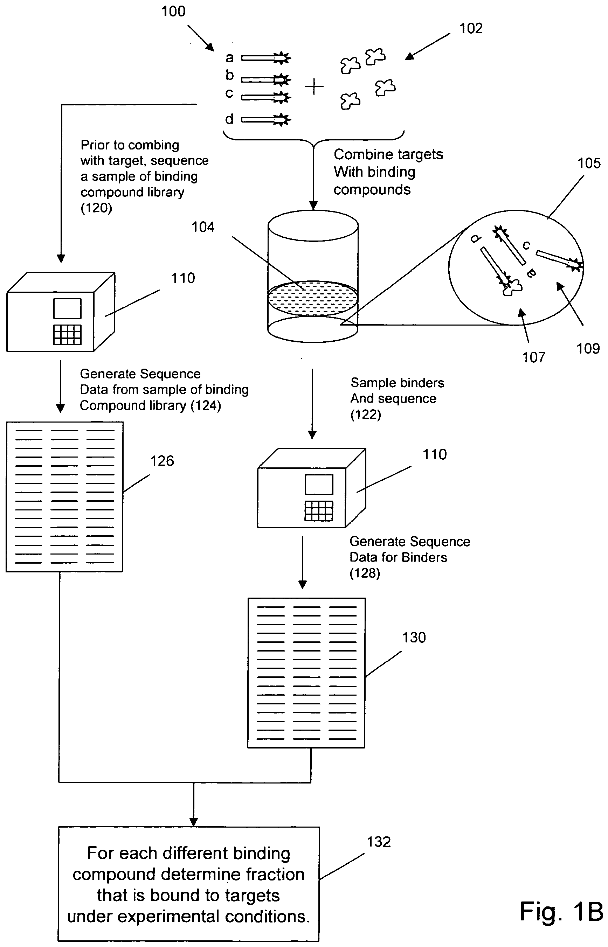

Fig. 1B illustrates diagrammatically a work flow of an alternative embodiment for measuring the binding strengths of candidate nucleic acid-encoded binding compounds. Prior to forming reaction mixture (104) with nucleic acid-encoded binding compounds (100) and target molecules (102), a sample of the binding compound library is taken and its members' encoding nucleic acids are sequenced (120), using high throughput sequencing device (110). Hosts expressing binding compounds are readily separated from non-expressing hosts using antibodies specific for constant regions, e.g. goat anti-kappa chain antibody for isolating phage expressing human Fab fragments, as discussed more fully below. As mentioned above, the sample is large enough to ensure that all of the different encoding nucleic acids of the candidate binding compounds are determined with high probability. The output of such sequencing (124) is a table of sequence reads for binding compound library (126). In one embodiment, where equimolar amounts of binding compounds are added to reaction mixture (104), the number of sequence reads for each different binding compound is substantially the same. After such sample is taken, reaction mixture (104) is formed and allowed to reach an equilibrium condition with respect to free and bound binding compound, after which a sample is taken (122) of only those candidate binding compounds that are bound to target (i.e. only binders are sampled). The sequences of the encoding nucleic acids of such binders are then determined (128) using a conventional high throughput sequencing device (110) to give a table of sequence reads (130) of the encoding nucleic acids of the binders. The data in Tables (126) and (130) are then used to calculate (132) the fraction or ratio of each candidate binding compound that is bound to target in reaction mixture (104). In one embodiment, such a fraction or ratio may be calculated by simply enumerating the sequence reads of each candidate binding compound in each Table and then taking the ratio of the numbers. As exemplified below, conventional techniques are used to determine relative amounts of candidate bind compounds to be combined with the one or more ligands in binding reactions so that the above sequence information can be obtained and converted into measures related to affinities.

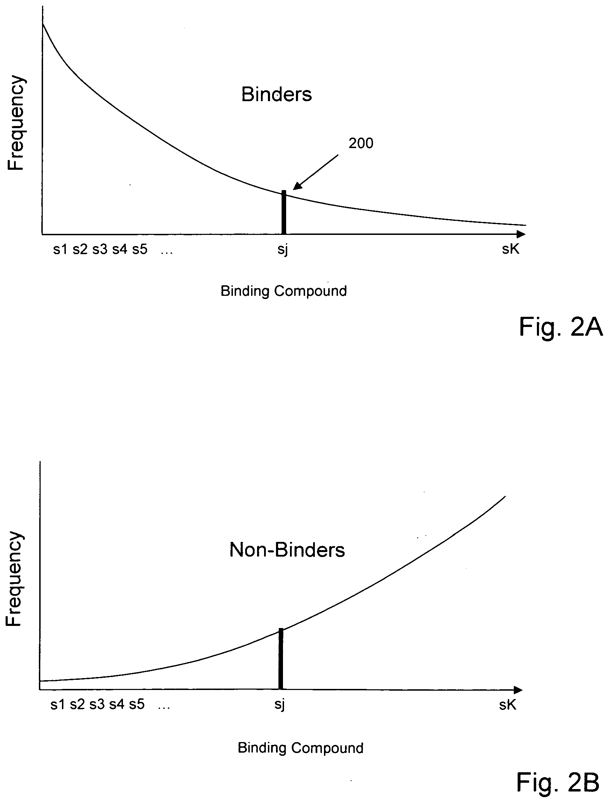

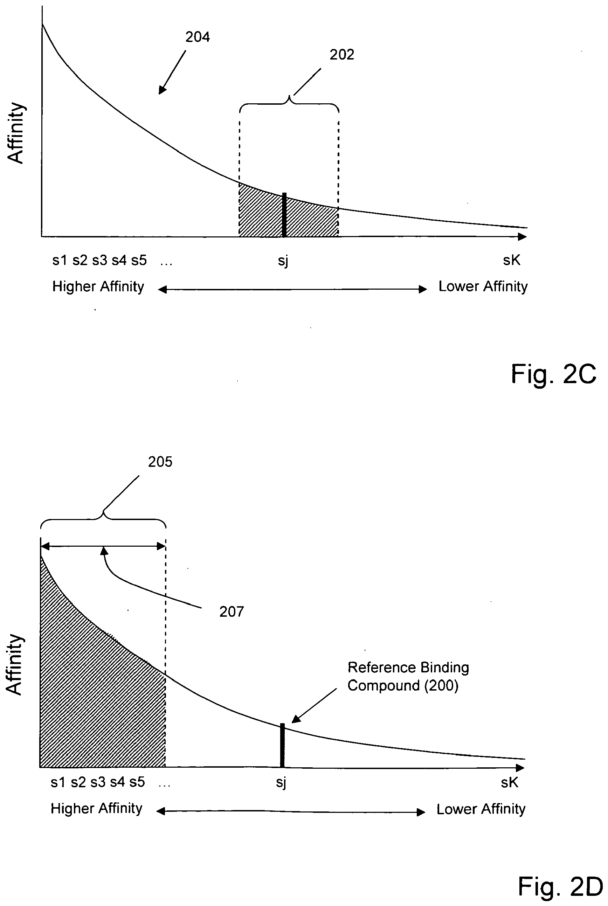

Nucleic acids encoding the binders and non-binders from the samples may be sequenced using any of a variety of commercially available high-throughput DNA sequence analyzers (110), as described more fully below, to generate sequence data for binders (112) and non-binders (114). Conventional sample preparation procedures are employed that take into account the particular format of the candidate binding compounds. That is, binding compounds may be phage display, ribosome display, retroviral display, or the like, and may require different steps to extract their nucleic acids and to prepare them for sequencing. The results of the sequence analysis are typically at least two tabulations of sequences corresponding to the binders (116) and non-binders (118). From such data, relationships between sequence frequency of binding compound and binding compound type may be shown, as illustrated in Figs. 2A-2B, or between affinity and binding compound type may be shown, as illustrated in Figs. 2C-2D. (Likewise, similar relationships may be observed for nonbinders.) Sequences of the encoding nucleic acids of the binders (Fig. 2A) and non-binders (Fig. 2B) may be ordered in accordance with their frequencies in the two tabulations (i.e. tables (116) and (118) of Fig. 1). Fig. 2A shows such an ordering (s₁, s₂, s₃, ... sₖ) for binders, and Fig. 2B shows a corresponding ordering for non-binders. In accordance with the invention, sufficient numbers of sequences are obtained so that the frequencies of the sequences are reliable statistics of the actual populations in equilibrium under the given conditions. Relative affinities of the nucleic acid-encoded binding compounds may be inferred from this data, as shown in Figs. 2C-2D. In the case where a standard (or equivalently a reference or a wild type) binding compound (200) (having sequence sⱼ) is present, its position on the graph may be identified, as well as those of "bio-similars" (202) (i.e., in this case, sequences encoding binding compounds with equivalent affinity to the antigen) and "bio-betters" (204) (i.e., in this case, sequences encoding binding compounds with superior affinity to the antigen). From relationships, as shown in Fig. 2C, binding compounds having different encoding sequences may be selected having the same or superior binding properties that a standard (or wild type or reference) binding compound. Binding compounds from among these alternatives that encode different amino acid sequences may be further selected to optimize other properties of interest, including cross-reactivity, specificity, stability, solubility, immunogenicity, or the like. The relationships illustrated in Figs. 2A-2C may also be equivalently represented in the form of a heat map (illustrated in Fig. 2F), where for example, an array of values (e.g. affinity) as a function of (usually) two parameters (e.g. amino acid or residue position and mutant residue) is represented by colors, shades of gray, or some other visual indicator of intensity. For example, a heat map may consist of an array of affinity values for combinations of (i) amino acid positions in a variable region of a light chain of an antibody and (ii) type of amino acid. The affinity values may be represented by colors across a spectrum from violet (highest affinity) to red (lowest affinity) or by grays along a gray scale from black (highest affinity) to white (lowest affinity). Binding compounds encoded by nucleic acids of set (202) that have different amino acid sequences from the reference binding compound are of particular interest, particularly (but not solely) when amino acid differences occur in the CDRs. As used herein, such binding compounds are referred to as "neutral binding compounds" for (i) their equivalence in binding affinity to a selected pre-existing, or reference, binding compound, and (ii) their amino acid sequences that are different from the reference binding compound. This latter characteristic permits selection for improvements of other properties of interest, e.g. increased solubility, increased stability, reduced cross-reactivity, reduced immunogenicity, or the like.. In some embodiments of the invention, binding compounds having improved solubility, reduced cross-reactivity, and/or reduced immunogenicity are selected from a set of neutral binding compounds. In one embodiment, neutral binding compounds comprise a set of binding compounds whose affinities are within forty percent of the affinity of a reference binding compound (i.e. either within forty percent higher than or within forty percent lower than the affinity of the reference binding compound). In another embodiment, neutral binding compounds comprise a set of binding compounds whose affinities are within ten percent of the affinity of a reference binding compound. In another embodiment, neutral binding compounds comprise a set of binding compounds whose affinities are within five percent of the affinity of a reference binding compound. In a further embodiment, neutral binding compounds comprise up to 100 candidate binding compounds having the closest affinity to that of a reference binding compound, but differing in amino acid sequence from the reference compound. In a further embodiment, neutral binding compounds comprise up to 1000 candidate binding compounds having the closest affinity to that of a reference binding compound, but differing in amino acid sequence from the reference compound. In some embodiments of the invention, the above method may be used to identify neutral binding compounds with respect to a reference compound using the following steps: (a) reacting under binding conditions a ligand with a library of candidate binding compounds and a reference binding compound, each candidate binding compound and the reference binding compound consisting of or being encoded by a nucleotide sequence; (b) determining the nucleotide sequences of binding compounds forming complexes with the ligand; (c) determining the nucleotide sequences of binding compounds free of ligand; (d) ordering the nucleotide sequences of the binding compounds in accordance with the affinities of their respective binding compounds for the ligand, wherein the affinities are determined by comparing the number of times a nucleotide sequence is identified among binding compounds forming complexes with the ligand and the number of times the same nucleotide sequence is identified among the binding compounds free of the ligand; and (e) identifying among the ordering of nucleotide sequences those nucleotide sequences whose orderings are adjacent to the ordering of a nucleotide sequence encoding the reference binding compound. In one embodiment, adjacent nucleic acids are nucleic acids encoding binding compounds whose affinities are within ten percent of the affinity of a reference binding compound (i.e. either within ten percent higher than or within ten percent lower than the affinity of the reference binding compound).

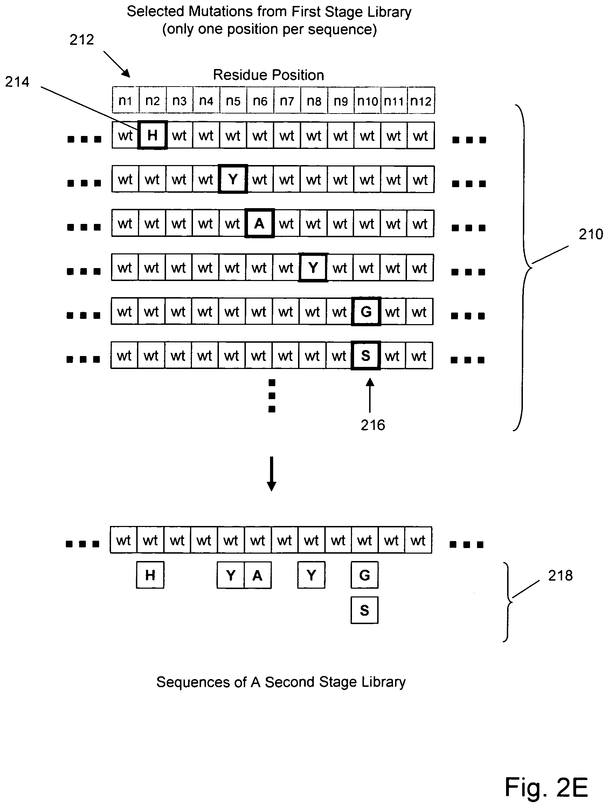

In some embodiments of the invention, after binding compounds are ordered with respect to affinity for a desired antigen, e.g. as shown in Fig. 2D, mutations of a subset (205) of the high affinity binding compounds, or high affinity and neutral binding compounds, may be used to construct a new, or second stage, library, which can be used to select for further improvements, where the further improvements may be for still higher affinity, reduced immunogenicity, increased stability, or the like. The size of the subset in a particular embodiment may be determined by how many of the top affinity binding compounds are used for obtaining mutants, which is simply how many of the left hand-most sequences (207) are used, as illustrated in Fig. 2D. In other embodiments, mutations may be selected by other criteria, e.g. avoidance of particular residues, such as hydrophobic residues, or the like. In some embodiments, such a second stage library may be constructed based on the selected mutations as illustrated in Fig. 2E. List (210) shows portions of sequences (positions (212) n₁ through n₁₂) from members of a first stage library in the subset of binders that have higher affinities for a predetermined antigen than that of a reference binding compound. In a full first stage library, for example, member sequences vary only at one residue at a time; thus, for the top most sequence (showing "H" at n₂), only position n₂ would have different amino acids substituted and at no other positions. In one embodiment, for a second stage library, a fully combinatorial library is constructed from the mutations that individually have an affinity higher than that of a reference binding compound. Thus, for the mutations of Fig. 2E, a second stage library would include sequences obtained by independently substituting the mutations of the first stage subset at the indicated positions. This is, for n₂ H and the wild type amino acid would be substituted; for n₅ Y and the wild type amino acid would be substituted; for n₆ A and the wild type amino acid would be substituted; and for n₁₀ G, S and the wild type amino acid would be substituted, so that in all 2x2x2x2x3 (= 48) distinct members would be obtained.

In some embodiments of the invention, the number of candidate binding compounds under consideration may be reduced in cases where improvements are sought to a pre-existing binding compound, i.e., a standard or reference binding compound, such as pre-existing known antibody, such as a known therapeutic antibody. For example, for a pre-existing antibody where the amino acid sequence of both its scaffold and binding regions are known, limited, or subregions of such sequences may be assessed for the effect of every possible single amino acid change in such subregions only and an estimate the combinatorial effects of multiple mutations may be obtained by adding the measured effects of the individual single amino acid changes. In other embodiments, such a process may be generalized by assessing the effect of every possible two-way amino acid change in the subregion, with an increased number of mutants requiring assessment. Such methods require a much smaller library to assess the effects of all the possible amino acid changes. For example, in the former embodiment, in a limited region of 50 amino acid positions, only 50 x 20 = 1000 mutants would need to be analyzed. In addition the assumption of achieving independent effects from multiple mutations used in combination is a good approximation when working with a small number of positions (<20).

Radioligand studies may be used to assess the above binding compound, but such studies usually are run serially, using multiple protein variants against a single radioligand in separate reactions, because the variant proteins are difficult to distinguish one from another. One could run multiple binding studies simultaneously, in the same reaction vessel, if the variant receptors were readily distinguishable from one another. This situation can be achieved using any of a number of viral, phage, or ribosome display formats, as described below. In these systems the variant receptors are displayed in low numbers (≤10 copies/particle) on the surface of viral, phage or ribosome particles. In these situations the specific nucleic acid that encoded the variant receptor is contained within the cognate virus/phage/ribosomal particle (also referred to herein as a nucleic acid-encoded binding compound). This allows easy identification of each specific protein variant by sequencing the nucleic acid that is attached to it. If this principle is applied to binding experiment described above, one can easily measure the binding affinities of large numbers of protein variants simultaneously by running an equilibrium binding assay using a virus/phage/ribosomal library (collection of variants) against a single ligand (either bound to a substrate or in solution). After equilibrium has been reached the bound receptors (phage/virus/ribosomal particles) can be collected by recovering the ligand molecules via immunoprecipitation or substrate recovery and the unbound receptors can be recovered from the supernatant. These two samples of phage/virus/ribosome particles can then be sequenced on a massively parallel fragment sequencer (as described below) to determine each clone's contribution to the bound and free pools of receptors. From this sequence information the bound percentage of each receptor in the library can be calculated. Those receptors with the highest percentage of bound phage/virus/ribosomes will have the highest affinities and those with the lowest bound percentages will have the lowest affinities. Using a single ligand concentration near the dissociation constant, K_{D}, of the parent protein, it is possible to rank the affinities every protein variant for a given ligand. If the parent molecule is encoded in the library, then the affinities of all of the variants in the library can be assessed relative to the parent protein, which serves as an internal standard or reference. If the ligand is in great excess in the binding reaction (so its unbound concentration does not change appreciably during the binding reaction) and several binding reactions are run using varying ligand concentrations, then one is able to use non-linear regressions or equivalent calculation to rapidly calculate the K_{D} for every variant in the population from the equation K_{D}=[A][B]/[AB]. In some embodiments employing protein display systems, such as phage display libraries, affinities may be estimated as follows based on tabulated sequences of nucleic acids encoding binding compounds. Multiple reactions are set up, e.g. in wells of a microtiter plate, or the like, such that the reactions contain a dilution series of ligand, i.e. a series of lower and lower concentrations or amounts of ligand adsorbed or attached to a solid support, such as the surface of a microwell wall, magnetic bead, or the like. To each reaction is added a fixed number of display organism, such as aliquots of a phage display library, and the reactions are allowed to go to equilibrium. After equilibrium has been reached, bound and free display organisms are harvested and binding-compound encoding nucleic acids are amplified in separate polymerase chain reactions (PCRs) to determine the reaction in which the concentration, or amount, of ligand results in about equal amounts of display organism bound to ligand and free. Under such conditions, affinities of the binding compounds may be estimated as ratios of bound binding compound (determined by counting encoding nucleic acids) and unbound binding compound (also determined by counting encoding nucleic acids). In some embodiments, a similar operation may be used to estimate affinities of binding compounds of a library relative to that of a reference binding compound (as used herein, such values are referred to as "relative affinities" with respect to a selected reference compound). As above, multiple reactions are set up with a dilution series of immobilized ligand. To each reaction is added a fixed amount of reference binding compound (e.g. a single phage displaying the reference binding compound) and the reactions are allowed to go to equilibrium. After equilibrium has been reached, bound and free display organisms are harvested and their encoding nucleic acids are amplified in separate PCRs to determine the reaction in which the concentration, or amount, of ligand results in about equal amounts of reference binding compound bound to ligand and free of ligand. The determined reaction provides conditions for carrying out library-based binding reactions so that ratios of binders to nonbinders for each library member can be computed and compared to that of a reference binding compound to give a measure of the relative affinity of such member to a ligand.

This information may be used to create an engineering diagram of the binding site in question (such as a heat map) which can be used to direct the engineering of any amino acid position within the binding site. Thus variants that have higher binding affinities than the parent molecule can be combined to markedly increase the protein's affinity for its ligand. Variants with the same binding affinities as the parent molecule can be used to increase the molecule's stability or solubility, reduce its immunogenicity or alter its serum half-life. In addition if the same protein library is run against multiple ligands, then the resulting heat maps can be overlaid to identify variants that differentially affect the binding of the ligands. Finally variants that reduce the binding affinity of the protein for its ligand(s) can be identified. In general these variants are to be avoided in future engineering projects, but in certain situations reducing a protein's activity by lowering its affinity for its ligand may be desirable.

### Selection for Improved Physical, Chemical and Biological Characteristics

In some embodiments, the 2D maps, or heat maps, described above display relative affinity among candidate binding compounds as a function of position (where amino acid substitutions are made) and the kind of amino acid(s) substituted. For providing binding compounds with increased affinity, mutations (i.e. candidate binding compounds identified by row and column positions) that have the highest relative affinities are identified so that a subset of candidate binding compounds may be identified in which those mutations are fixed. Members of the subset may then be further assayed to identify mutants with other improved characteristics, along with the higher relative affinities. Also, such an initially identified subset may be used to generate further libraries. For example, a new library may be created from the above subset by fixing the amino acids conferring increased affinity and varying amino acids in the remaining positions, or a fraction of the remaining positions, or in additional positions in the same sequence that were not varied in the original library.

Virtually every member of the originally identified subset will have increased affinity relative to wild-type and some will be substantially higher. To increase the solubility of a molecule, neutral mutations (with respect to binding affinity) are identified from the 2D map that replace uncharged surface residues with charged ones and the resultant molecules will have increased solubility. If it is desired to decrease pI (so increase half-life), the 2D map can be used to find neutral mutations in which positively charged surface residues are replaced with negatively or neutrally charged residues. In addition replacing neutrally charged surface residues with negatively charged residues will achieve the same goal. In some embodiments, the above may be implemented in accordance with the invention to increase the solubility of a selected nucleic acid-encoded binding compound (i.e. reference binding compound) without loss of affinity for a ligand by the following steps: (a) reacting under binding conditions one or more ligands with a library of candidate binding compounds, each candidate binding compound being comprised of or encoded by a nucleotide sequence; (b) determining the nucleotide sequences of the candidate binding compounds forming complexes with the one or more ligands; (c) determining for each candidate binding compound an affinity based on a number of nucleotide sequences of binding compounds forming a complex to its total number in the library; and (d) selecting at least one candidate binding compound from a subset of candidate binding compounds (i) whose affinity is equal to or greater than that of the selected nucleic acid-encoded binding compound and (ii) whose encoding nucleic acid encodes at least one charged amino acid residue in place of a neutral or hydrophobic amino acid residue occurring in the selected nucleic acid-encoded binding compound, thereby providing a nucleic acid-encoded binding compound with increased solubility with respect to the reference binding compound without loss of affinity. In one embodiment, the library of step (a) may be a first stage library as described above; or step (a) may be carried out in two phases using a first stage library in a first phase and a second stage library as described above in a second phase. In another embodiment, a second stage library as described above may be used in step (d).

In some embodiments, the method of the invention may be used to obtain a binding compound with equivalent or better affinity as that of a reference binding compound, but which has superior stability with respect to selected destabilizing agents. A subset of candidate compounds identified as described above based on affinity is separated into at least two portions. Members of a first portion are compared to members of a second portion after members of the latter portion have been treated with a destabilizing agent (heat, low pH, proteases, or the like). That is, both portions originated from the same starting subset of candidate binding compounds, except that the members of the second portion are subjected to a destabilizing agent. In other words, its members form a "stressed" library. The candidate binding compounds from such a library that lose binding affinity after being "stressed" contain destabilizing residues. A goal is to identify mutants that bind the antigen at least as well or better than wild type in the "stressed" library. It is expected that several stabilizing mutations could be combined to dramatically increase the stability of the molecule, for example, by forming a second-stage library from such mutants and conducting a second round of selection. In some embodiments, the above may be implemented in accordance with the invention to increase stability of a selected nucleic acid-encoded binding compound (i.e. reference binding compound) without loss of affinity for a ligand by the steps of: (a) treating a library of candidate binding compounds with a destabilizing agent to form a treated library of candidate binding compounds, each candidate binding compound being comprised of or encoded by a nucleotide sequence; (b) reacting under binding conditions one or more ligands with the treated library of candidate binding compounds; (c) determining the nucleotide sequences of the candidate binding compounds forming complexes with the one or more ligands; (d) determining for each candidate binding compound an affinity based on a ratio of a number of nucleotide sequences of binding compounds forming a complex to its total number in the treated library; and (e) selecting at least one candidate binding compound from a subset of candidate binding compounds whose affinity is equal to or greater than that of the selected nucleic acid-encoded binding compound (that is, the reference binding compound), thereby providing a nucleic acid-encoded binding compound with increased stability with respect to the reference binding compound without loss of affinity. As above, in one embodiment, the library of step (a) may be a first stage library as described above; or step (a) may be carried out in two phases using a first stage library in a first phase and a second stage library as described above in a second phase. In another embodiment, a second stage library as described above may be used in step (d).

In some embodiments, for example, for binding compounds expressed in phage display systems, exemplary conditions for stressing a subset include (i) exposing phage to elevated temperatures, e.g. in the range of 50-70°C for a period of time, e.g. in the range of 15-30 minutes; (ii) exposing phage to low pH, e.g. pH in the range of 1-4, for a period of time, e.g. in the range of 15-30 minutes; (iii) exposing phage to various proteases at various activities over a range for a period of time, e.g. 15-30 minutes, or 1-4 hours, or 1 hour to 24 hours, depending on the protease and specific activity. Exemplary proteases for stability testing include, but are not limited to, serum proteases; trypsin; chymotrypsin; cathepsins, including but not limited to cathepsin A and cathepsin B; endopeptidases, such as, matrix metalloproteinases (MMPs) including, but not limited to, MMP-1, MMP-2, MMP-9; or the like.

In some embodiments, immunogenicity may be altered after the locations of immunogenic peptides within the protein of interest are identified. Immunogenicity, which can be a problem even with fully human antibodies, can make pharmacokinetic assessment more difficult, reduce safety, and inhibit effectiveness, e.g. by stimulating neutralizing host antibodies. Identifying peptides derived from a protein of interest that can stimulate helper T-cells (the first step in the immunogenicity cascade) has been described (J. Immunol. Methods, 281(1-2): 95-108 (2003)). Once identified, the 2D genetic map can be used to identify neutral substitutions which may be incorporated into new peptide that is re-tested in the immunogenicity assay. Given the completeness of the 2D map, multiple variant peptides can be selected for testing. Selection of peptide variants having the lowest immunogenicity yields a molecule with similar binding affinity as that of the parent, but with reduced immunogenicity. In some embodiments, an immunogenicity assay is employed that provides a predictive measure of immunogenicity, such as ability to stimulate T-cells in vitro (Stickler et al, Toxicol. Sci., 77(2): 280-289 (2004); Harding et al, mAbs, 2(3): 256-265 (2010); or the like. Several companies provide services for determining immunogenic peptides based on their ability to be bound by MHC class II molecules, e.g., Antitope in Cambridge, England. In some embodiments of the invention, relative immunogenicity is determined; that is, immunogenicity of a test binding compound is compared to that of a reference binding compound. In some embodiments, "reduced immunogenicity" as used herein means that the immunogenicity measured relative to a candidate binding compound is less than that of a reference binding compound. As mentioned above, immunogenicity may be measured by the proliferative response elicited in peripheral blood mononuclear cells by exposure to a test compound. In one embodiment (following Stickler et al, cited above), test compounds comprise a set of overlapping peptides derived from a candidate binding compound for binding to MHC molecules, e.g. each having a size in the range of from 10 to 20 amino acids. Monocyte-derived dendritic cells and CD4+ T cells for the assays are obtained by conventional procedures. Briefly (for example), monocytes are purified by adherence to plastic in AIM V medium (Gibco/Life Technologies, Baltimore, MD). Adherent cells are cultured in AIM V media containing 500 units/ml of recombinant human IL-4 (Endogen, Wobum, MA) and 800 units/ml recombinant human GM-CSF (Endogen) for 5 days. On day 5, recombinant human IL-1α (Endogen) and recombinant human TNF-α (Endogen) are added at 50 units/ml and 0.2 units/ml, respectively. On day 7, the fully matured dendritic cells are treated with 50 µg/ml mitomycin c (Sigma Chemical Co., St. Louis, MO) for 1h at 37°C. Treated dendritic cells are dislodged with 50 mM EDTA in PBS, washed in AIM V media, counted, and resuspended in AIM V media at 2 x 10⁵ cells/ml. CD4+ T cells are purified by negative selection from frozen aliquots of PBMC using Cellect CD4 columns (Cedarlane, Toronto, Ontario, Canada) or Dynabeads (Dynal Biotech, Oslo, Norway). CD4+ T cell populations are typically >80% pure and >95% viable as judged by Trypan blue (Sigma Chemical Co.) exclusion. CD4+ T cells are resuspended in AIM V media at 2 x 10⁶ cells/ml. CD4+ T cells and dendritic cells are plated in round bottomed 96-well format plates at 100 µl of each cell mix per well. The final cell number per well is 2 x 10⁴ dendritic cells and 2 x 10⁵ CD4+ T cells. Peptide is added to a final concentration of about 5 µg/ml in 0.25-0.5% DMSO. Control wells contain DMSO without added peptide. Each peptide is tested in duplicate. Cultures are incubated at 37°C in 5% CO₂ for 5 days. On day 5, 0.5 µCi of triturated thymidine (NEN/DuPont, Boston, MA) is added to each well. On day 6, the cultures are harvested onto glass fiber mats using a TomTec manual harvester (TomTec, Hamden, CT) and then processed for scintillation counting. Proliferation is assessed by determining the average CPM value for each set of duplicate wells (TriLux Beta, Wallac, Finland).

In some embodiments of the invention, a method of reducing the immunogenicity of a selected nucleic acid-encoded binding compound (i.e. reference binding compound) without loss of affinity comprises the following steps: (a) reacting under binding conditions one or more ligands with a library of candidate binding compounds, each candidate binding compound being comprised of or encoded by a nucleotide sequence; (b) determining the nucleotide sequences of the candidate binding compounds forming complexes with the one or more ligands; (c) determining for each candidate binding compound an affinity based on a ratio of a number of nucleotide sequences of binding compounds forming a complex to its total number in the library; (d) selecting at least one candidate binding compound from a subset of candidate binding compounds (i) whose affinity is equal to or greater than that of the selected nucleic acid-encoded binding compound and (ii) whose encoding nucleic acid encodes at least one amino acid residue different from that of the selected nucleic acid-encoded binding compound at the same location(s) and reduces the immunogenicity of such candidate binding compound relative to that of the selected nucleic acid-encoded binding compound. As above, in one embodiment, the library of step (a) may be a first stage library as described above; or step (a) may be carried out in two phases using a first stage library in a first phase and a second stage library as described above in a second phase. In another embodiment, a second stage library as described above may be used in step (d).

In some embodiments, the method of the invention may be used to obtain a binding compound with equivalent or better affinity to a target antigen as that of a reference binding compound, but that has reduced cross reactivity, or in some embodiments, increased cross reactivity, with selected substances, such as ligands, proteins, antigens, or the like, other than the substance or epitope for which a reference binding compound is specific, or is design to be specific for. In regard to the latter, a candidate therapeutic antibody may be more successfully tested in animal models if the antibody reacted with both its human target and the corresponding target of the animal model, e.g. mouse. Thus, in some embodiments, the method of the invention may be employed to increase cross reactivity with selected substances, such as corresponding animal model targets. In other embodiments, the method of the invention is employed to reduce cross reactivity of a candidate therapeutic antibody, for example, to reduce potential side effects in a patient. As above, a subset of candidate compounds is identified based on affinity (i.e. having equivalent or higher affinity than that of the reference compound). Candidate compounds from the subset may then be combined with one or more substances other than the target antigen in one or more binding reactions (e.g. each at different phage concentrations) to determine the affinities of such candidate binding compounds to such substances. The choice of substances may vary widely, and may include tissues, cell lines, selected proteins, tissue arrays, protein microarrays, or other multiplex displays of potentially cross reactive compounds. Guidance for selecting such antibody cross reaction assays may be found in the following exemplary references: Michaud et al, Nature Biotechnology, 21(12): 1509-1512 (2003); Kijanka et al, J. Immunol. Methods, 340(2): 132-137 (2009); Predki et al, Human Antibodies, 14(1-2): 7-15 (2005); Invitrogen Application Note on Protoarray^{™} Protein Microarray (2005); and the like. In such binding reactions, nucleic acids encoding binders and non-binders from the subset are determined in accordance with the invention, thereby providing statistically significant values of dissociation constants of each candidate binding compound of the subset for the one or more selected substances for which cross reactivity information was sought. As above, knowledge of the sequences of low-cross reactivity mutants may be used to generate a second stage library to identify binding compounds with further reduced cross reactivity with the selected substances.

In some embodiments, the above may be implemented in accordance with the invention to identify one or more binding compounds with reduce cross reactivity with a selected set of substances compared to that of a reference binding compound without loss of affinity for a ligand. Such method may be carried out by the steps of: (a) reacting under binding conditions one or more substances with a subset of candidate binding compounds, each member of the subset having equivalent of greater affinity for a ligand than that of a reference compound; (b) determining the nucleotide sequences of the candidate binding compounds forming complexes with the one or more substances; (c) determining for each candidate binding compound an affinity based on a ratio of a number of nucleotide sequences of binding compounds forming a complex to its total number in the subset; and (d) selecting at least one candidate binding compound from the subset of candidate binding compounds whose affinity is equal to or less than that of the reference binding compound, thereby providing a nucleic acid-encoded binding compound with reduced cross reactivity for the one or more substances with respect to the reference binding compound without loss of affinity. Likewise, a method may be implemented for obtaining a binding compound with increased reactivity to a selected substance or compound or epitope by substituting step (d) with the following step: selecting at least one candidate binding compound from the subset of candidate binding compounds whose ratio is equal to or greater than that of the reference binding compound.

### Protein Display Systems

Features of any peptide or protein display system are: 1. Tight linkage between the expressed proteins and their encoding nucleic acid; and 2. Expression of the protein in a format that allows it to be assayed and separated based on some biochemical activity (for example, binding strength, susceptibility to enzymatic action, or the like). For the purposes of this discussion, protein display systems can be separated into two groups based on the number of displayed proteins per display unit, either polyvalent or monovalent. The polyvalent display systems such as yeast display (references 1 and 2 below), mammalian display systems (references 3 and 4 below) and bacterial display systems (reference 5) express the gene(s) of interest (often diverse antibody libraries) as proteins tethered to the cell surface by means of a membrane anchor, similar to a native surface immunoglobulin found on the plasma membrane of normal B-cells. DNA encoding the library clones is transformed into the cell type of interest such that each cell receives at most one clone from the library. The resultant population of cells will each express tens to tens of thousands of copies of a single protein clone on their cell surfaces. This population of cells can then be exposed to limiting amounts of fluorescently labeled target antigen and the best binding clones will bind the most antigen and they can be identified and isolated using a fluorescence-activated cell sorter (FACS). Unfortunately accurate quantitation in polyvalent display systems is complicated by cooperative binding effects (avidity) between the multiple copies of the displayed molecule on the same cell (reference 6). This problem is especially pronounced if the antigen is polyvalent (TNF, IgG) or bound to a cell surface (e.g. CD 20).

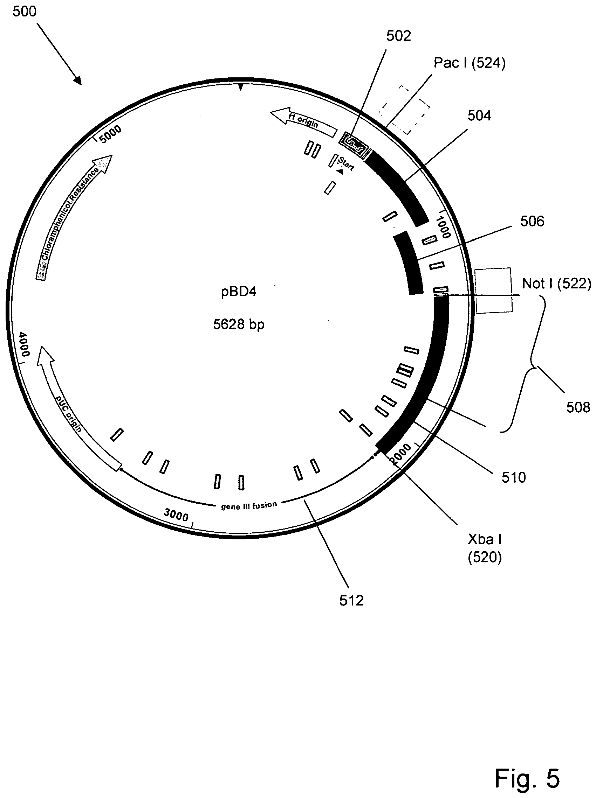

Many of the viral and phage-based protein display systems are also polyvalent in nature, but the display units are too small to detect on the FACS, so accurate quantitation is even more difficult. These systems also suffer from avidity problems if multiple binding compounds are expressed simultaneously on the same phage particle. Under such conditions it is difficult to determine whether an observed binding strength is due to the combined effect of two expressed binding compounds versus the effect of a single very high affinity binding compound. Such avidity problems may be minimized by regulating the expression of candidate binding compound in a host using conventional techniques. In one embodiment in which a phage display system expresses Fab fragments, e.g. as disclosed in Fig. 5, regulation of Fab expression is adjusted so that the fraction of phage expression Fab is in the range of from about .002 to .001, or in the range of about .001 to .0005.

The monovalent phage (reference 7) and viral (reference 8) systems, along with the ribosome display systems (references 9 and 10) express an average of ≤1 molecule of the displayed molecule per display unit. These systems yield accurate measurements of the true affinity of the binding site in question for each clone in the library. Generally these systems are used to display large, diverse libraries of binding elements. Small subpopulations of clones are then selected from these libraries based on their increased ability to bind the target antigen relative to other members of the library. After selection (often multiple rounds of selection) the resultant clones are isolated and characterized (e.g. as disclosed in U.S. patent 7,662,557. This is a good strategy for isolating initial binders to a given target antigen from a very large and diverse library, but is not an efficient method for mapping a single protein binding site for the purposes of protein engineering. To achieve this goal one would like to characterize the effect of every possible engineering change and then design and construct an optimized binding site based on: affinity, stability, cross-reactivity, immunogenicity, circulating half-life, manufacturing yield, etc. Therefore it would be desirable to analyze the binding strength of every member of a saturated, single substitution library of the binding site in question. The above protein display techniques are disclosed in the following exemplary references, which are incorporated herein by reference: (1) Wittrup, KD; Current Opinion in Biotechnology 12: 395-399 (2001) (Protein engineering by cell-surface display); (2) Lauren R. Pepper, Yong Ku Cho, Eric T. Boder and Eric V. Shusta; Combinatorial Chemistry & High Throughput Screening 11: 127-134 (2008); (3) Yoshiko Akamatsu, Kanokwan Pakabunto, Zhenghai Xu, Yin Zhang, Naoya Tsurushita; Journal of Immunological Methods 327: 40-52 (2007); (4) Chen Zhou, Frederick W. Jacobsen, Ling Cai, Qing Chen and Weyen David Shen; mAbs 2(5): 1-11 (2010); (5) Patrick S Daugherty; Current Opinion in Structural Biology 17:474-480 (2007) (Protein engineering with bacterial display); (6) Clackson and Lowman (editors), Phage Display (2009); (7) Hennie R Hoogenboom, Andrew D Griffiths, Kevin S Johnson, David J Chiswell, Peter Hudson and Greg Winter; Nucleic Acids Research 19(15): 4133-4137 (1991); (8) Francesca Gennari, Luciene Lopes, Els Verhoeyen, Wayne Marasco, Mary K. Collins; Human Gene Therapy 20: 554-562 (2009); (9) Christiane Schaffitzel, Jozef Hanes, Lutz Jermutus, Andreas Pluckthun; Journal of Immunological Methods 231: 119-135 (1999) (ribosome display); (10) Robert A Irving, Gregory Coia, Anthony Roberts, Stewart D Nuttall, Peter J Hudson; Journal of Immunological Methods 248: 31-45 (2001) (ribosome display); (11) Arvind Rajpal, Nurten Beyaz, Lauric Haber, Guido Cappuccilli, Helena Yee, Ramesh R Bhatt, Toshihiko Takeuchi, Richard A Lerner, Roberto Crea; PNAS 102 (24): 8466-71(2005). Some of the above techniques are also disclosed in the following patents: 7,662,557; 7,635,666; 7,195,866; 7,063,943; 6,916,605; and the like.

Further protein display systems for use with the invention include baculoviral display systems, adenoviral display systems, lentivirus display systems, retroviral display systems, SplitCore display systems, as disclosed in the following references: Sakihama et al, PLosOne 3(12): e4024 (2008); Makela et al, Combinatorial Chemistry & High Throughput Screening, 11: 86-98 (2008); Urano et al, Biochem. Biophys. Res Comm., 308: 191-196 (2003); Gennari et al, Human Gene Therapy, 20: 554-562 (2009); Taube et al, PLosOne, 3(9): e3181 (2008); Lim et al, Combinatorial Chemistry & High Throughput Screening, 11:111-117 (2008); Urban et al, Chemical Biology, 6(1): 61-74 (2011); Buchholz et al, Combinatorial Chemistry & High Throughput Screening, 1: 99-110 (2008); Walker et al, Scientific Reports, 1(5): (14 June 2011); and the like.

In some embodiments, the invention employs conventional phage display systems for improving one or more properties of a antibody binding compound, particularly a preexisting antibody binding compound. Unlike prior applications of display technologies, which employ repeated cycles of selection, washing, elution and amplification, to identify individual phage from a large library, e.g. >10⁸-10⁹ clones, in the present invention, a single equilibrium binding reaction is created using a relatively small and focused library, e.g. 10³-10⁴ clones, or in some embodiments 10⁴-10⁵ clones, after which binder and non-binders are analyzed by large-scale sequencing. From such analysis, subsets are selected and, optionally, further selected based on other properties of interest, such as, solubility, stability, lack of immunogenicity, and the like. Factors affecting such equilibrium reactions are well-known in the art and include: the number of phage to include in the reaction, the stringency of the reaction mixture; the number of target molecules to include in the reaction; presence or absence of blocking agents, such as, bovine serum albumin, gelatin, casein, or the like, to reduce nonspecific binding; the length and stringency of a wash step to separate non-binders; the nature of an elution step to remove binders from the target molecules; the format of target molecules used in the reaction, which, for example, may be bound to a solid support or derivatized with a capture agent, e.g. biotin, and free in solution; the phage protein into which candidate binding compounds are inserted; and the like. In some embodiments, target molecules, such as proteins, are purified and directly immobilized on a solid support such as a bead or microtiter plate. This enables the physical separation of bound and unbound phage simply by washing the support. Numerous supports are available for this purpose, including modified affinity resins, glass beads, modified magnetic beads, plastic supports, and the like. Useful supports are those that have low background for nonspecific phage binding and that present the target molecules in a native configuration and at a desirable concentration.

In some embodiments, a nucleic acid-encoded binding compound is an antibody fragment expressed by a phage. In one embodiment, such phage is a filamentous bacteriophage and the antibody fragment is expressed as part of a coat protein. In particular, such phage may be a member of the Ff class of bacteriophages. In a further embodiment, the host of such filamentous bacteriophage is E. coli. In another embodiment, a phagemid-helper phage system is used for displaying antibody fragments. Phagemids may be maintained as plasmids in a host bacteria and phage production induced by further infection with a helper phage. Exemplary phagemids include pComb3 and its related family members, e.g. disclosed in Barbas et al, Proc. Natl. Acad. Sci., 88: 7978-7982 (1991), and pHEN1 and its related family members, e.g. disclosed in Hoogenboom et al, Nucleic Acids Research, 19: 4133-4137 (1991); and U.S. patent 5,969,108; 6,806,079; 7,662,557; and related patents. In a particular embodiment, an antibody fragment is expressed as a fusion protein with phage coat protein g3p.

### Libraries of Nucleic Acid-Encoded Binding Compounds

As mentioned above, a feature of the invention is the use of focused libraries from which reliable binding statistics can be obtained from a binding reaction. In some embodiments this eliminates the need for successive cycles of selection, elution, and amplification, as required in conventional approaches. The size of such focused libraries of candidate binding compounds is influenced by at least two factors: the scale of sequencing required for analyzing binders and nonbinders and the difficulty of synthesizing polynucleotides that encode library members. That is, the larger the library of candidate compounds and the higher the degree of confidence desired in the binding statistics of each compound both require that more binders and nonbinders be sequenced. Likewise, a larger library of candidate compounds means a greater number of polynucleotides need to be synthesized. Thus, particular applications may involve conventional design choices between scale of implementation and cost. In some embodiments, focused libraries are obtained by varying amino acids in a limited number of locations one or two at a time within a pre-existing binding compound, which may be the same as, or equivalent to, a reference binding compound. Preferably amino acids are varied at different positions one at a time. Thus, for example, members of a library of candidate binding compounds may have nucleotide sequences identical to that encoding the pre-existing binding compound except for a single codon position. At that position, each member will have a codon different from that of the pre-existing binding compound. Such libraries may include members having an amino acid deletion at such location and may not necessarily include members with every possible codon at such location. Libraries may contain members corresponding to such substitutions (and deletions) at each of a set of amino acid locations within the pre-existing binding compound. The locations may be contiguous or non-contiguous. In some embodiments, the number of locations where codons are varied are in the range of from 1 to 500; in some embodiments, the number of such locations are in the range of from 1 to 250; in other embodiments, the number of such locations are in the range of from 10 to 100; and in still other embodiments, the number of such locations are in the range of from 10 to 250. A pre-existing binding compound may be any pre-existing antibody for which sequence information is available (or can be obtained). Typically, a pre-existing binding compound is a commercially important binding compound, such as an antibody drug, for which one desires to modify one or more properties, such as solubility, immunogenicity, reduction of cross reactivity, increase in stability, aggregation resistance, or the like, as discussed above. In one embodiment, the locations where codons are varied comprise the V_{H} and V_{L} regions of the antibody, including both codons in framework regions and in CDRs; in another embodiment, the locations where codons are varied comprise the CDRs of the heavy and light chains of the antibody, or a subset of such CDRs, such as solely CDR1, solely CDR2, solely CDR3, or pairs thereof. In another embodiment, locations where codons are varied occur solely in framework regions; for example, a library may comprise single codon changes solely from a reference binding compound solely in framework regions of both V_{H} and V_{L} numbering in the range of from 10 to 250. In another embodiment, the locations where codons are varied comprise the CDR3s of the heavy and light chains of the antibody, or a subset of such CDR3s. In another embodiment, the number of locations where codons of V_{H} and V_{L} encoding regions are varied are in the range of from 10 to 250, such that up to 100 locations are in framework regions. In another embodiment, nucleic acid encoded binding compounds are derived from a pre-existing binding compound, such as a pre-existing antibody. Exemplary pre-existing binding compounds include, but are not limited to, antibody-targeted drugs or antibody-based drugs such as adalimumab (Humira), bevacizumab (Avastin), cetuximab (Erbitux), efalizumab (Raptiva), infliximab (Remicade), panitumumab (Vectubix), ranibuzumab (Lucentis), rituximab (Rituxan), trastuzumab (Herceptin), and the like.

In some embodiments, the above codon substitutions are generated by synthesizing coding segments with degenerate codons. The coding segments are then ligated into a vector, such as a replicative form of a phage, to form a library. Many different degenerate codons may be used with the present invention, such as those shown in Table I.

**Table I.**

| Exemplary Degenerate Codons | | | |
|---|---|---|---|
| Codon* | Description | Stop Codons | Number |
| NNN | All 20 amino acids | TAA, TAG, TGA | 64 |
| NNK or NNS | All 20 amino acids | TAG | 32 |
| NNC | 15 amino acids | none | 16 |
| NWW | Charqed, hydrophobic | TAA | 16 |
| RVK | Charged, hydrophilic | none | 12 |
| DVT | Hydrophilic | none | 9 |
| NVT | Charged, hydrophilic | none | 12 |
| NNT | Mixed | none | 16 |
| WC | Hydrophilic | none | 9 |
| NTT | Hydrophobic | none | 4 |
| RST | Small side chains | none | 4 |
| TDK | Hydrophobic | TAG | 6 |

| | | | |
|---|---|---|---|
| * Symbols follow the IUB code: N=G/A/T/C, K=G/T, S=G/C, W=A/T, R=A/G, V=G/A/C, and D=G/A/T. | | | |

In some embodiments, the size of binding compound libraries used in the invention varies from about 1000 members to about 1 x 10⁵ members; in some embodiments, the size of libraries used in the invention varies from about 1000 members to about 5 x 10⁴ members; and in further embodiments, the size of libraries used in the invention varies from about 2000 members to about 2.5 x 10⁴ members. Thus, nucleic acid libraries encoding such binding compound libraries would have sizes in ranges with upper and lower bounds up to 64 times the numbers recited above.

### Nucleic Acid Sequencing Techniques

As mentioned above, a variety of DNA sequence analyzers are available commercially to determine the nucleotide sequences of binder and non-binders in accordance with the invention. Commercial suppliers include, but are not limited to, 454 Life Sciences, Helicos, Life Technologies Corp., Illumina, Inc. (which produces sequencing instruments using Solexa-based sequencing techniques), Pacific Biosciences, and the like. Also, DNA sequencing techniques under commercial development may be used for implementing the invention, e.g. techniques disclosed in the following references. Rothberg et al, Nature, 475: 348-352 (2011); Rothberg et al, U.S. patent publication 2009/0026082; Anderson et al, Sensors and Actuators B Chem., 129: 79-86 (2008); Pourmand et al, Proc. Natl. Acad. Sci., 103: 6466-6470 (2006); Rothberg et al, U.S. patent publication 2010/0137143; Meller et al, U.S. patent publication 2009/0029477; and the like. The use of particular types DNA sequence analyzers is a matter of design choice, where a particular analyzer type may have performance characteristics (e.g. long read lengths, high number of reads, short run time, cost, etc.) that are particularly suitable for the experimental circumstances and binding compounds being analyzed. DNA sequence analyzers and their underlying chemistries have been reviewed in the following references, for their guidance in selecting DNA sequence analyzers: Bentley et al, Nature, 456: 53-59 (2008)(describing Solexa-based sequencing); Kircher et al, Bioessays, 32: 524-536 (2010); Shendure et al, Science, 309: 1728-1732 (2005); Margulies et al, Nature, 437: 376-380 (2005); Metzker, Nature Reviews Genetics, 11: 31-46 (2010); Hert et al, Electrophoresis, 29: 4618-4626 (2008); Anderson et al, Genes, 1: 38-69 (2010); Fuller et al, Nature Biotechnology, 27: 1013-1023 (2009); and the like. Generally, nucleic acids of binding compounds are extracted and prepared for sequencing in accordance with instructions of a DNA sequence analyzer's instructions.

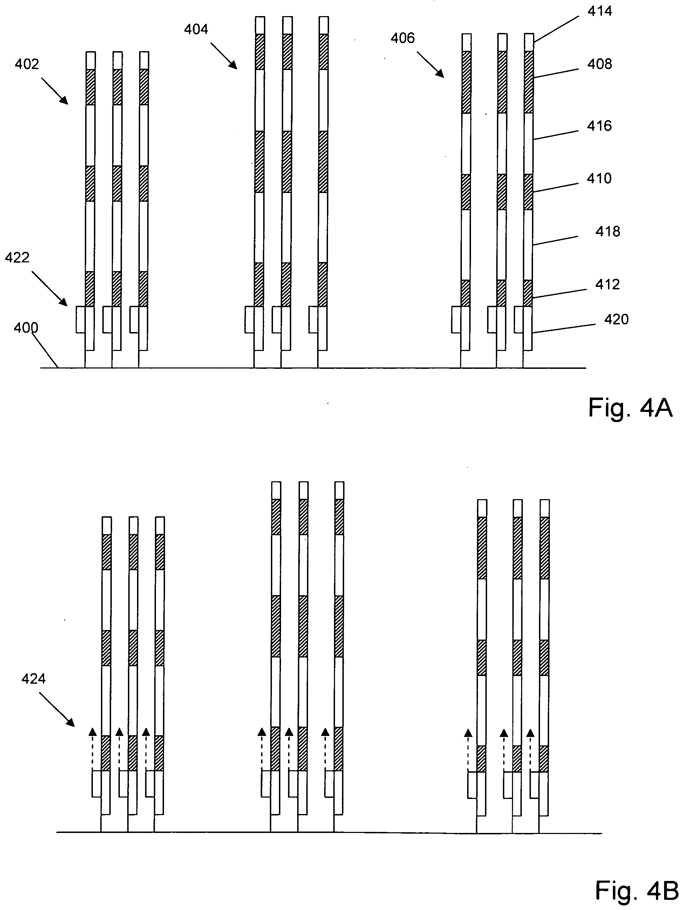

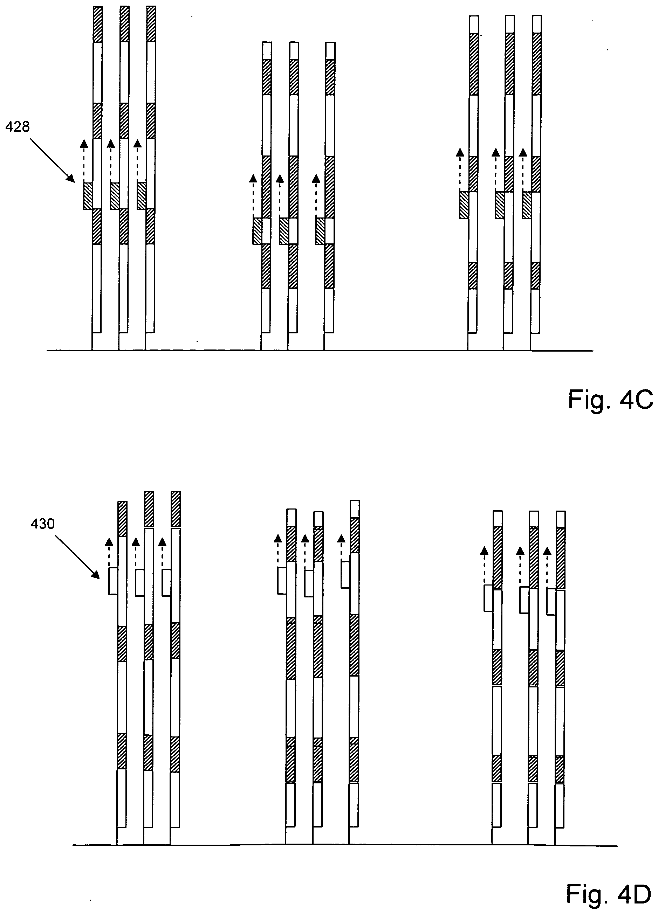

In one embodiment, a limited read length sequencing technique, such as that disclosed by Bentley et al (cited above), is employed to identify discrete regions of a longer encoding nucleic acid. As used herein, the term "limited read length" in reference to a sequencing method means that the longest sequence of nucleotides identified in a single sequencing reaction comprises less than about one hundred nucleotides. As described above, nucleic acids of binders and non-binders are sequenced to obtain structural information about a target molecule. Depending on the nature of the binding compounds employed, the sequencing task can vary widely. Generally, the number, sizes and separations of the regions where amino acids are varied in binding compounds will determine how much sequence information is required for identification. Typically, limited read length sequencing methods cannot provide enough sequence information from a single sequencing reaction for identification. However, in the case where binding compounds are antibodies whose CDRs are varied, complete identification may be obtained with a limited read length method if at least three sequencing reactions are performed on a single nucleic acid. Accordingly, in one embodiment of the invention, nucleic acids corresponding to CDRs from antibody-based binding compounds are serially analyzed by performing at least three sequencing reactions on the same target nucleic acid. The method is illustrated in Figs. 4A-4D. As shown in Fig. 4A, nucleic acids extracted from binding compounds are amplified to form clonal populations (402, 404, and 406, for example) on solid support (400), e.g. using bridge PCR as disclosed by Bentley et al (cited above). Dark regions (408, 410 and 412) represent CDR-encoding regions of the nucleic acids of the respective antibody-based binding compounds, which are used to identify the binding compounds. Light-colored regions (414, 416, 418 and 420) encode the antibody scaffold regions and are the same among all the binding compounds. Thus, a limited read length method may be employed by carry out three separate primer-based sequencing reactions where each reaction uses a primer that anneals to a scaffold region adjacent to a different CDR encoding region of the same target nucleic acid. As shown in Fig. 4A, primer (422) anneals to the scaffold region adjacent to the CDR encoding region proximal to solid surface (400). The same primer will anneal at the same position in all of the different target nucleic acids (402, 404 and 406). After annealing primer (422), a limited read length sequencing reaction is performed (424) and the sequences of the adjacent CDRs are obtained, as represented in Fig. 4B. The extended primers are then removed and the process is repeated using primer (428), illustrated in Fig. 4C, and again with primer (430) as illustrated in Fig. 4D. The three sequences form an ordered set that completely identifies the binding compound whose encoding nucleic acid is analyzed. In some embodiments, the above method of identifying an antibody-based binding compound using a limited read length sequencing technique may be implemented with the following steps: (a) forming spatially separate clonal populations of each nucleic acid encoding an antibody-based binding compound on a surface, each nucleic acid having identical scaffold encoding regions and a first discrete CDR-encoding region, a second discrete CDR-encoding region, and a third discrete CDR-encoding region; (b) performing a limited read length primer-based sequencing reaction from a first primer annealed to a first scaffold, or framework, encoding region adjacent to the first discrete CDR-encoding region to obtain a first read of the nucleic acid; (c) performing a limited read length primer-based sequencing reaction from a second primer annealed to a second scaffold, or framework, encoding region adjacent to the second discrete CDR-encoding region to obtain a second read of the nucleic acid; (d) performing a limited read length primer-based sequencing reaction from a third primer annealed to a third scaffold, or framework, encoding region adjacent to the third discrete CDR-encoding region to obtain a third read of the nucleic acid; and (e) identifying the antibody-based binding compound from the first, second and third reads of the nucleic acid.

### EXAMPLE

### Construction of an A vastin-Based Binding Compound Library

Listed below are the sequences of the heavy chain variable region and the light chain variable region of the humanized antibody Avastin (bevacizumab), Presta et al, Cancer Research, 57: 4593-4599 (1997). Together these two proteins form the high affinity binding site for VEGF that gives Avastin its efficacy against many solid tumors. It is known from structural studies on this and many other antibodies that the key amino acids involved in physically binding its ligand, VEGF, are located within the "CDR" regions highlighted by underlining.

To gain a complete functional map of all the possible single amino acid substitutions in the binding site of Avastin, two libraries of variant molecules need to be constructed. A complete single amino substitution library of the Avastin heavy chain will include 820 proteins (41 positions x 20 amino acids). A complete single amino substitution library of the Avastin light chain will include 540 proteins (27 positions x 20 amino acids). Each of these libraries may be constructed in a number of ways, including the use of oligonucleotide-directed mutagenesis to create pools of variant molecules that each carry a randomization codon (NNN) at a different position within the CDR sequences. In this example the Avastin heavy chain library would be composed of 41 pools of genes each containing a randomization codon (NNN) at a different position in the Avastin heavy chain CDRs. This would yield a redundant library of 2624 genes (41 positions x 64 codons) for the heavy chain library. These 41 pools of sequences containing 2624 V_{H} genes each differing from the parent by at most by a single codon can be cloned into a standard phagemid display vector either as a Fabs or single-chain Fv's in conjunction with the wild type light chain. (Note that each pool contains a member that is wild type and numerous silent wild type variants also exist within the larger population). Likewise the 27 pools of Avastin V_{L} genes containing 1728 members each differing from the parent by at most one codon can be cloned into the same vector in conjunction with the wild type heavy chain gene to create the Avastin light chain library.

Once created and confirmed, these two libraries can be transformed into an appropriate bacterial strain to create stably transformed bacterial cell libraries. In this situation each antibody variant is carried in a separate bacterial cell. These two populations of cells can then be induced to produce phage particles by infecting them with a helper phage. The helper phage carries the phage genes that are missing in the phagemid and allows the cells to start producing one type of phage per cell. Infecting a population of cells carrying the full spectrum of single amino acid variants will produce a full spectrum of phage each carrying a variant Fab or scFv at its tail which was encoded by the single stranded DNA in its attached genome. The two libraries can then be harvested and used in two ways. First their diversity can be efficiently characterized using a massively parallel fragment sequencer (454, Illumina, ABI) to make sure that full spectrum libraries have been created. Next the libraries can be titred and set up in equilibrium binding assays with several concentrations of the VEGF ligand fused to a tag useful for immunoprecipitation (i.e. Fc-fusion). For maximum resolution the differing concentrations of the ligand should center around the K_{D} of the parent antibody and should vary in 2-10 fold increments. Care must be taken to scale the reactions to assure that the antigen is in large excess, so its free concentration will not be reduced during the binding reaction. These reactions are incubated until equilibrium is reached (for example, 22°C for 24 hr in conventional binding reaction mixture). Once equilibrium has been reached, the two types of phage can be separated. The phage that are bound to the soluble antigen can be immunoprecipitated. using a reagent that is specific for the ligand fusion, like protein A or an anti-Fc antibody. The unbound phage can then be isolated from the depleted supernatant from each reaction, e.g. by precipitating unbound binding-compound-expressing phage with anti-kappa chain antibody, anti-lambda chain antibody, anti-C_{H}1 antibody, anti-tag antibody, such as a myc tag, polyhistidine tag, or the like. Specifically, in one embodiment, human Fab-bearing phage may be isolated either by binding goat anti-kappa chain antibody followed by capture with protein G coated beads, or by binding biotinylated anti-kappa chain antibody followed by capture with streptavidin-coated beads. Alternatively to the above, binders and non-binders may be identified in a competitive binding reaction where, for example, library binding compounds compete with a reference binding compound for binding to an immobilized antigen, either by displacing previously bound reference compound or by being combined with antigen and reference compound at the same time. Guidance for carrying out such reactions is found in Wild, editor, The Immunoassay Handbook, 3rd Edition (Elsevier, 2008), and like references. The V-region segments from all of the variants from the two samples from each reaction can then be amplified via PCR to serve as substrates for one of the massively parallel fragment sequencing platforms. Using the Illumina sequencer as an example, the bound and the free fractions from a single binding reaction of the Avastin heavy chain library would be sequenced in individual lanes of a flow cell. Each lane should yield between 10 and 30 million V-region sequences. Thus each of the 2641 genes in the Avastin library would be sequenced an average of 10,000 times between the two lanes. This is a very large number indicating that multiple reactions could be looked at simultaneously given a proper indexing scheme. Numbers for each clone from each lane of the flow cell can be tabulated and the two data sets can be combined to calculate percentage binding for each gene. These percentages can then be used to accurately rank the affinities of all of the genes in the library. As mentioned earlier there are two types of wild-type genes in the library: true wild types and silent mutations of wild-type. In some CDR sequencing schemes, only the latter will be available for use as internal standards, since wild-type CDRs dominate each library. This data can then be used to create an engineering heat map describing the effect of every possible mutation in the binding site and its effect on the protein's binding affinity for its ligand. This data can further be compiled into a plasticity map that codes each amino acid in the binding site for its ability to be changed without reducing the protein's binding affinity. Each amino acid that is actually playing an important role in the binding reaction will be highly intolerant to change, whereas amino acid positions that are not involved in the binding reaction should be much more tolerant to change.
Avastin V_{H} (SEQ 10 NO: 1)
Avastin V_{L} (SEQ ID NO: 2)

A library of such Avastin-based binding compounds was constructed as follows. Prior to inserting a mixture of synthetic segments to create a phagemid library, two phagemids were constructed with similar structures to the pHEN1 phagemid disclosed by Hoogenboom et al (cited above). Each of the phagemids includes a sequence that encodes an Fab fragment; however, one phagemid is engineered to accept variable light chain encoding sequences with a wild type heavy chain (i.e. the light chain library) and the other phagemid is engineered to accept variable heavy chain encoding sequences with a wild type light chain (i.e. the heavy chain library). The starting phagemid for both constructs was a pBCSK⁺ (Stratagene, San Diego, CA). Since the phagemids are grown in a conventional f⁺ E. coli host (XL1 Blue, Stratagene), a bacterial leader sequence (MKYLLPTAAAGLLLLAAQPAMA (SEQ ID NO: 3)) was added to each of the above sequences for the Avastin V_{H} and V_{L} regions. In addition, the following ribosome binding site sequences were appended to the 5' ends of the nucleotide sequences encoding the VH and VL regions: CTAGTTAATTAAaggaggagcaggg (SEQ ID NO: 4) for the light chain (designated Fab-12 LC) and CTAGGCGGCCGCaggaggagcaggg (SEQ ID NO: 5) for the heavy chain (designated Fab-12 HC). The Lac promoter and polylinker elements of the pBCSK vector were rearranged and gene III was inserted, after which the light and heavy chain encoding regions were inserted in several steps to give a construct pBD4 (500), illustrated in Fig. 5 for the phagemid encoding the wild type Fab. Codons for the Fab regions were selected for expression in the E. coli host. The light chain library is constructed from the appropriate phagemid by swapping in the synthetic light chain library polynucleotides to a Pac I (524)-NotI (522) segment engineered into the construct. Likewise, the heavy chain library is constructed from the appropriate phagemid by swapping in the synthetic heavy chain library polynucleotides into a NotI (522)-Xba I (520) segment engineered into the construct. The resulting phagemid (500) for the heavy chain library has in sequence Lac promoter (502), and segments encoding the wild type light chain variable region (504), light chain constant region (506), heavy chain variable region (508), heavy chain constant region (510) and gene III fusion partner (512). Library sequences are expressed by infecting the host carrying the phagemids with a conventional helper phage (e.g. M13K07, New England Biolabs).

### Definitions

Unless otherwise specifically defined herein, terms and symbols of nucleic acid chemistry, biochemistry, genetics, and molecular biology used herein follow those of standard treatises and texts in the field, e.g. Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Abbas et al, Cellular and Molecular Immuology, 6th edition (Saunders, 2007).

"Antibody" or "immunoglobulin" means a protein, either natural or synthetically produced by recombinant or chemical means, that is capable of specifically binding to a particular antigen or antigenic determinant, which may be a target molecule as the term is used herein. Antibodies, e.g. IgG antibodies, are usually heterotetrameric glycoproteins of about 150,000 daltons, composed of two identical light (L) chains and two identical heavy (H) chains, as illustrated in Fig. 3. Each light chain is linked to a heavy chain by one covalent disulfide bond, while the number of disulfide linkages varies between the heavy chains of different immunoglobulin isotypes. Each heavy and light chain also has regularly spaced intra-chain disulfide bridges. Each heavy chain has at one end a variable domain (V_{H}) followed by a number of constant domains. Each light chain has a variable domain at one end (V_{L}) and a. constant domain at its other end; the constant domain of the light chain is aligned with the first constant domain of the heavy chain, and the light chain variable domain is aligned with the variable domain of the heavy chain, as illustrated in Fig. 3. Typically the binding characteristics, e.g. specificity, affinity, and the like, of an antibody, or a binding compound derived from an antibody, are determined by amino acid residues in the V_{H} and V_{L} regions, and especially in the CDR regions. The constant domains are not involved directly in binding an antibody to an antigen. Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), e.g., IgG" IgG₂, IgG₃, IgG₄, IgA₁, and IgA₂. "Antibody fragment", and all grammatical variants thereof, as used herein are defined as a portion of an intact antibody comprising the antigen binding site or variable region of the intact antibody, wherein the portion is free of the constant heavy chain domains (i.e. CH2, CH3, and CH4, depending on antibody isotype) of the Fc region of the intact antibody. Examples of antibody fragments include Fab, Fab', Fab'-SH, F(ab')₂, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv (scFv) molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific or multivalent structures formed from antibody fragments. The term "monoclonal antibody" (mAb) as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each mAb is directed against a single determinant on the antigen. In addition to their specificity, the monoclonal antibodies are advantageous in that they can be synthesized by hybridoma culture or by bacterial, yeast or mammalian expression systems, uncontaminated by other immunoglobulins.

"Binding compound" means a compound that is capable of specifically binding to a particular target molecule or group of target molecules. Examples of binding compounds include antibodies, receptors, transcription factors, signaling molecules, viral proteins, lectins, nucleic acids, aptamers, and the like, e.g. Sharon and Lis, Lectins, 2nd Edition (Springer, 2006); Klussmann, The Aptamer Handbook: Functional Oligonucleotides and Their Applications (John Wiley & Sons, New York, 2006). As used herein, "antibody-based binding compound" means a binding compound derived from an antibody, such as an antibody fragment, including but not limited to, Fab, Fab', F(ab')₂, and Fv fragments, or recombinant forms thereof. In some embodiments, an antibody-based binding compound comprises a scaffold or framework region of an antibody and CDR regions of an antibody.

"Complementary-determining region" or "CDR" means a short sequence (up to 13 to 18 amino acids) in the variable domains of immunoglobulins. The CDRs (six of which are present in IgG molecules) are the most variable part of immunoglobulins and contribute to their diversity by making specific contacts with a specific antigen, allowing immunoglobulins to recognize a vast repertoire of antigens with a high affinity, e.g. Beck et al, Nature Reviews Immunology, 10: 345-352 (2010). Several numbering schemes, such as the Kabat numbering scheme, provide conventions for describing amino acid locations of CDRs within variable regions of immunoglobulins.

"Complex" as used herein means an assemblage or aggregate of molecules in direct or indirect contact with one another. In some embodiments, "contact," or more particularly, "direct contact" in reference to a complex of molecules, or in reference to specificity or specific binding, means two or more molecules are close enough so that attractive noncovalent interactions, such as Van der Waal forces, hydrogen bonding, ionic and hydrophobic interactions, and the like, dominate the interaction of the molecules. In such an embodiments, a complex of molecules is stable in that under assay conditions, the presence of the complex is thermodynamically favorable. As used herein, "complex" may refer to a stable aggregate of two or more proteins, which is equivalently referred to as a "protein-protein complex." A complex may also refer to an antibody bound to its corresponding antigen. Complexes of particular interest in the invention are protein-protein complexes and antibody-antigen complexes. As noted above, various types of noncovalent interactions may contribute to antibody binding of antigen, including electrostatic forces, hydrogen bonds, van der Waals forces, and hydrophobic interactions. The relative importance of each of these depends on the structures of the binding site of the individual antibody and of the antigenic determinant. The strength of the binding between a single combining site of an antibody and an epitope of an antigen, which can be determined experimentally by equilibrium dialysis (e.g. Abbas et al (cited above)), is called the affinity of the antibody. The affinity is commonly represented by a dissociation constant (K_{d}), which describes the concentration of antigen that is required to occupy the combining sites of half the antibody molecules present in a solution of antibody. A smaller K_{d} indicates a stronger or higher affinity interaction, because a lower concentration of antigen is needed to occupy the sites. For antibodies specific for natural antigens, the K_{d} usually varies from about 10⁻⁷ M to 10⁻¹¹ M. Serum from an immunized individual will contain a mixture of antibodies with different affinities for the antigen, depending primarily on the amino acid sequences of the CDRs.

"Ligand" means a compound that binds specifically and reversibly to another chemical entity to form a complex. Ligands include, but are not limited to, small organic molecules, peptides, proteins, nucleic acids, and the like. Of particular interest are protein-ligand complexes, which include protein-protein complexes, antibody-antigen complexes, enzyme-substrate complexes, and the like.

"Phage display" is a technique by which variant polypeptides are displayed as fusion proteins to at least a portion of a coat protein on the surface of phage, e.g., filamentous phage, particles. A utility of phage display lies in the fact that large libraries of randomized protein variants can be rapidly and efficiently selected for those sequences that bind to a target molecule with high affinity. Display of peptide and protein libraries on phage has been used for screening millions of polypeptides for ones with specific binding properties. Polyvalent phage display methods have been used for displaying small random peptides and small proteins through fusions to either gene II or gene VIII of filamentous phage. Wells and Lowman, Curr. Opin. Struct. Biol., 3:355-362 (1992), and references cited therein. In monovalent phage display, a protein or peptide library is fused to a gene III or a portion thereof, and expressed at low levels in the presence of wild type gene III protein so that phage particles display one copy or none of the fusion proteins. Avidity effects are reduced relative to polyvalent phage so that selection is on the basis of intrinsic ligand affinity, and phagemid vectors are used, which simplify DNA manipulations. Lowman and Wells, Methods: A companion to Methods in Enzymology, 3:205-0216 (1991).

"Phagemid" means a plasmid vector having a bacterial origin of replication, e.g., ColE1, and a copy of an intergenic region of a bacteriophage. The phagemid may be used on any known bacteriophage, including filamentous bacteriophage and lambdoid bacteriophage. The plasmid will also generally contain a selectable marker for antibiotic resistance. Segments of DNA cloned into these vectors can be propagated as plasmids. When cells harboring these vectors are provided with all genes necessary for the production of phage particles, the mode of replication of the plasmid changes to rolling circle replication to generate copies of one strand of the plasmid DNA and package phage particles. The phagemid may form infectious or non-infectious phage particles. This term includes phagemids, which contain a phage coat protein gene or fragment thereof linked to a heterologous polypeptide gene as a gene fusion such that the heterologous polypeptide is displayed on the surface of the phage particle.

"Phage" or "phage vector" means a double stranded replicative form of a bacteriophage containing a heterologous gene and capable of replication. The phage vector has a phage origin of replication allowing phage replication and phage particle formation. The phage is preferably a filamentous bacteriophage, such as an M13, f1, fd, Pf3 phage or a derivative thereof, or a lambdoid phage, such as lambda, 21, phi80, phi81, 82, 424, 434, etc., or a derivative thereof. particle.

"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references Dieffenbach, editor, PCR Primer: A Laboratory Manual, 2nd Edition (Cold Spring Harbor Press, New York, 2003).

"Polypeptide" refers to a class of compounds composed of amino acid residues chemically bonded together by amide linkages with elimination of water between the carboxy group of one amino acid and the amino group of another amino acid. A polypeptide is a polymer of amino acid residues, which may contain a large number of such residues. Peptides are similar to polypeptides, except that, generally, they are comprised of a lesser number of amino acids. Peptides are sometimes referred to as oligopeptides. There is no clear-cut distinction between polypeptides and peptides. For convenience, in this disclosure and claims, the term "polypeptide" will be used to refer generally to peptides and polypeptides. The amino acid residues may be natural or synthetic.

"Protein" refers to a polypeptide, usually synthesized by a biological cell, folded into a defined three-dimensional structure. Proteins are generally from about 5,000 to about 5,000,000 daltons or more in molecular weight, more usually from about 5,000 to about 1,000,000 molecular weight, and may include posttranslational modifications, such acetylation, acylation, ADP-ribosylation, amidation, disulfide bond formation, farnesylation, demethylation, formation of covalent cross-links, formation of cystine, glycosylation, hydroxylation, iodination, methylation, myristoylation, oxidation, phosphorylation, prenylation, selenoylation, sulfation, and ubiquitination, e.g. Wold, F., Post-translational Protein Modifications: Perspectives and Prospects, pgs. 1-12 in Post-translational Covalent Modification of Proteins, B. C. Johnson, Ed., Academic Press, New York, 1983. Proteins include, by way of illustration and not limitation, cytokines or interleukins, enzymes such as, e.g., kinases, proteases, galactosidases and so forth, protamines, histones, albumins, immunoglobulins, scleroproteins, phosphoproteins, mucoproteins, chromoproteins, lipoproteins, nucleoproteins, glycoproteins, T-cell receptors, proteoglycans, and the like.

"Specific" or "specificity" in reference to the binding of one molecule to another molecule, such as a labeled target sequence for a probe, means the recognition, contact, and formation of a stable complex between the two molecules, together with substantially less recognition, contact, or complex formation of that molecule with other molecules. In some embodiments, "specific" in reference to the binding of a first molecule to a second molecule means that to the extent the first molecule recognizes and forms a complex with another molecule in a reaction or sample, it forms the largest number of the complexes with the second molecule. Preferably, this largest number is at least fifty percent. Generally, molecules involved in a specific binding event have areas on their surfaces or in cavities giving rise to specific recognition between the molecules binding to each other. Examples of specific binding include antibody-antigen interactions, enzyme-substrate interactions, formation of duplexes or triplexes among polynucleotides and/or oligonucleotides, receptor-ligand interactions, and the like. As used herein, "contact" in reference to specificity or specific binding means two molecules are close enough that weak noncovalent chemical interactions, such as Van der Waal forces, hydrogen bonding, base-stacking interactions, ionic and hydrophobic interactions, and the like, dominate the interaction of the molecules.

"Wild type" or "reference" or "pre-existing" in reference to a binding compound are used synonymously to means a compound which is being analyzed or improved in accordance with the method of the invention. That is, such a compound serves as a starting material from which variant polypeptides are derived through the introduction of mutations. A "wild type" sequence for a given protein is usually the sequence that is most common in nature, but the term is used more broadly here to include compounds that have been engineered. Similarly, a "wild type" gene sequence is typically the sequence for that gene which is most commonly found in nature, but the usage here includes genes that may have been engineered from a natural compound, e.g. a gene which has been engineered to consist of bacterial codons even though it encodes a human protein. Mutations may be introduced into a "wild type" gene (and thus the protein it encodes) through any available process, e.g. site-specific mutation, insertion of chemically synthesized segments, or other conventional means. The products of such processes are "variant" or "mutant" forms of the original "wild type" protein or gene. Exemplary reference (or wild type or pre-existing) sequences include antibody-targeted drugs or antibody-based drugs such as adalimumab (Humira), bevacizumab (Avastin), cetuximab (Erbitux), efalizumab (Raptiva), infliximab (Remicade), panitumumab (Vectubix), ranibuzumab (Lucentis), rituximab (Rituxan), trastuzumab (Herceptin), and the like.
## Claims

1. A method of identifying binding compounds that have equivalent or improved affinities to a ligand as that of a reference binding compound, the method comprising the steps of:
reacting under binding conditions a ligand with a library of binding compounds, the library comprising candidate binding compounds and a reference binding compound, wherein each candidate binding compound and the reference binding compound comprise a protein encoded by a nucleotide sequence and wherein each of the candidate binding compounds of the library is obtained by varying amino acids at one or two locations at a time with respect to the reference binding compound wherein each of the one or two locations is selected from 10 to 250 amino acid locations in the reference binding compound or one location is selected from 1 to 500 amino acid locations in the reference binding compound;
determining the nucleotide sequences of a sample of binding compounds forming complexes with the ligand;
determining the nucleotide sequences of a sample of binding compounds free of ligand or a sample of binding compounds from the library;
wherein each of said samples contains a number of binding compounds at least five times the size of the library;
ordering the nucleotide sequences of the binding compounds in accordance with the affinities of their respective binding compounds for the ligand, wherein the affinities are determined for each binding compound by comparing the number of times a nucleotide sequence is identified with the binding compound forming complexes with the ligand and the number of times the same nucleotide sequence is identified with the binding compound free of the ligand or the number of the same nucleotide sequence identified in the sample of binding compounds from the library; and
identifying among the ordering of nucleotide sequences those nucleotide sequences that encode candidate binding compounds having affinities that are equivalent to or greater than that of the nucleotide sequence encoding the reference binding compound.

2. The method of claim 1 wherein said step of reacting includes establishing an equilibrium condition with respect to said binding compounds forming complexes with said ligand and said binding compounds free of said ligand.

3. The method of claim 2 wherein said steps of determining nucleotide sequences of said binding compounds forming complexes with said ligand and determining nucleotide sequences of said binding compounds free of said one or more ligands each include sampling said binding compounds so that values of said numbers are statistically significant.

4. The method of claim 2 further including steps for identifying a binding compound with increased solubility with respect to said reference binding compound from among said candidate binding compounds that have affinities that are equivalent to or greater than that of said reference compound, the further steps comprising: selecting at least one binding compound from such candidate binding compounds whose encoding nucleic acid encodes at least one charged amino acid residue in place of a neutral or hydrophobic amino acid residue occurring at an equivalent position in said reference binding compound.

5. The method of claim 2 further including steps for identifying a binding compound with reduced immunogenicity with respect to said reference binding compound from among said candidate binding compounds that have affinities that are equivalent to or greater than that of said reference compound, the further steps comprising: selecting at least one binding compound from such candidate binding compounds whose encoding nucleic acid encodes at least one different amino acid residue in place of an amino acid residue occurring at an equivalent position in said reference binding compound and whose immunogenicity is reduced relative to that of said reference binding compound.

6. The method of claim 2 further including steps for identifying a binding compound with reduced cross reactivity to one or more substances with respect to said reference binding compound from among said candidate binding compounds that have affinities that are equivalent to or greater than that of said reference compound, the further steps comprising: (a) reacting under binding conditions one or more substances with such candidate binding compounds; (b) determining the nucleotide sequences of such candidate binding compounds forming complexes with the one or more substances; (c) determining for each such candidate binding compound a ratio of the number of nucleotide sequences of such candidate binding compound forming a complex with the one or more substances to its total number among such candidate binding compounds; and (d) selecting at least one candidate binding compound from such candidate binding compounds whose ratio is equal to or less than that of the reference binding compound, thereby providing a nucleic acid-encoded binding compound with reduced cross reactivity for the one or more substances with respect to the reference binding compound without loss of affinity.

7. The method of claims 1, 2, 3, 4, 5 or 6, wherein each of said binding compounds is an antibody or an antibody fragment expressed as a fusion protein in a protein display system.

8. The method of claim 7 wherein said protein display system is a phage display system.

9. The method of claim 7 wherein said sample from said library is obtained by capturing the antibodies or fragments thereof using an antibody that binds specifically to a CH1, kappa or lambda chain or using an antibody that binds specifically to a peptide tag thereon.

10. A method of identifying a binding compound with increased stability and with affinity to a ligand equivalent to or greater than that of a reference binding compound, the method comprising the steps of:
treating a library of candidate binding compounds and a reference binding compound with a destabilizing agent to form a treated library of binding compounds, wherein each of the candidate binding compounds of the library is obtained by varying amino acids at one or two locations at a time with respect to the reference binding compound wherein each of the one or two locations is selected from 10 to 250 amino acid locations in the reference binding compound or one location is selected from 1 to 500 amino acid locations in the reference binding compound and wherein each binding compound of the treated library is comprised of or encoded by a nucleotide sequence;
reacting under binding conditions a ligand with the treated library;
determining the nucleotide sequences of a sample of binding compounds forming complexes with the ligand;
determining the nucleotide sequences of a sample of binding compounds free of ligand or a sample of binding compounds from the library;
ordering the nucleotide sequences of the binding compounds in accordance with the affinities of their respective binding compounds for the ligand, wherein the affinities are determined for each binding compound by comparing the number of times a nucleotide sequence is identified with the binding compound forming complexes with the ligand and the number of times the same nucleotide sequence is identified with the binding compound free of the ligand or the number of the same nucleotide sequence identified in the sample of binding compounds from the library; and
identifying among the ordering of nucleotide sequences those nucleotide sequences that encode binding compounds having affinities that are equivalent to or greater than that of the nucleotide sequence encoding the reference binding compound.

11. The method of claim 10 wherein said binding compounds are antibodies or fragments thereof expressed by a protein display system.

12. The method of claim 11 wherein said destabilizing agent is pH in the range of from 1 to 4 or temperature in the range of from 50°C to 70°C.

13. The method of claim 11 wherein said destabilizing agent is a protease.

14. The method of claim 13 wherein said protease is selected from the group consisting of trypsin, chymotrypsin, cathepsin, and endopeptidase.

## Patentansprüche

1. Verfahren zur Identifizierung bindender Verbindungen, die äquivalente oder verbesserte Affinitäten zu einem Liganden gegenüber der einer bindenden Referenzverbindung aufweisen, wobei das Verfahren die folgenden Schritte umfasst:
Umsetzen eines Liganden unter Bindungsbedingungen mit einer Bibliothek von bindenden Verbindungen, wobei die Bibliothek bindende Kandidatenverbindungen und eine bindende Referenzverbindung umfasst, wobei jede bindende Kandidatenverbindung und die bindende Referenzverbindung ein von einer Nukleotidsequenz codiertes Protein umfassen und wobei die bindenden Kandidatenverbindungen der Bibliothek jeweils erhalten werden, indem Aminosäuren immer an einem oder zwei Orten gleichzeitig in Bezug auf die bindende Referenzverbindung variiert werden, wobei der eine Ort bzw. die zwei Orte jeweils aus 10 bis 250 Aminosäureorten in der bindenden Referenzverbindung ausgewählt ist bzw. sind oder ein Ort aus 1 bis 500 Aminosäureorten in der bindenden Referenzverbindung ausgewählt ist;
Bestimmen der Nukleotidsequenzen einer Probe von bindenden Verbindungen, die Komplexe mit dem Liganden bilden;
Bestimmen der Nukleotidsequenzen einer Probe von bindenden Verbindungen, die frei von Ligand sind, oder einer Probe von bindenden Verbindungen aus der Bibliothek;
wobei die Proben jeweils bindende Verbindungen in einer Anzahl enthalten, die wenigstens fünfmal so groß wie die Bibliothek ist;
Ordnen der Nukleotidsequenzen der bindenden Verbindungen gemäß den Affinitäten ihrer jeweiligen bindenden Verbindungen für den Liganden, wobei die Affinitäten für jede bindende Verbindung bestimmt werden, indem verglichen wird, wie viele Male eine Nukleotidsequenz damit identifiziert wird, dass die bindende Verbindung Komplexe mit dem Liganden bildet, und wie viele Male die gleiche Nukleotidsequenz damit identifiziert wird, dass die bindende Verbindung frei vom Liganden ist, oder der Anzahl der gleichen Nukleotidsequenz, die in der Probe von bindenden Verbindungen aus der Bibliothek identifiziert wird; und
Identifizieren derjenigen Nukleotidsequenzen unter der Anordnung von Nukleotidsequenzen, die bindende Kandidatenverbindungen mit Affinitäten codieren, die zu der der die bindende Referenzverbindung codierenden Nukleotidsequenz äquivalent oder größer als diese sind.

2. Verfahren nach Anspruch 1, wobei der Umsetzungsschritt Erstellen eines Gleichgewichtszustands in Bezug auf die bindenden Verbindungen, die Komplexe mit dem Liganden bilden, und die bindenden Verbindungen, die frei vom Liganden sind, umfasst.

3. Verfahren nach Anspruch 2, wobei die Schritte der Bestimmung von Nukleotidsequenzen der bindenden Verbindungen, die Komplexe mit dem Liganden bilden, und der Bestimmung von Nukleotidsequenzen der bindenden Verbindungen, die frei von dem einen bzw. den mehreren Liganden sind, jeweils ein Sampling der bindenden Verbindungen umfassen, so dass Werte für die Zahlen statistisch signifikant sind.

4. Verfahren nach Anspruch 2, weiter umfassend Schritte zur Identifizierung einer bindenden Verbindung mit erhöhter Löslichkeit in Bezug auf die bindende Referenzverbindung unter den bindenden Kandidatenverbindungen, die Affinitäten aufweisen, die zu der der bindenden Referenzverbindung äquivalent oder größer als diese sind, wobei die weiteren Schritte Folgendes umfassen: Auswählen wenigstens einer bindenden Verbindung aus solchen bindenden Kandidatenverbindungen, deren codierende Nukleinsäure wenigstens einen geladenen Aminosäurerest anstelle eines neutralen oder hydrophoben Aminosäurerests, der an einer äquivalenten Position in der bindenden Referenzverbindung vorliegt, codiert.

5. Verfahren nach Anspruch 2, weiter umfassend Schritte zur Identifizierung einer bindenden Verbindung mit reduzierter Immunogenität in Bezug auf die bindende Referenzverbindung unter den bindenden Kandidatenverbindungen, die Affinitäten aufweisen, die zu der der bindenden Referenzverbindung äquivalent oder größer als diese sind, wobei die weiteren Schritte Folgendes umfassen:
Auswählen wenigstens einer bindenden Verbindung aus solchen bindenden Kandidatenverbindungen, deren codierende Nukleinsäure wenigstens einen unterschiedlichen Aminosäurerest anstelle eines Aminosäurerests, der an einer äquivalenten Position in der bindenden Referenzverbindung vorliegt, codiert und deren Immunogenität relativ zu der der bindenden Referenzverbindung reduziert ist.

6. Verfahren nach Anspruch 2, weiter umfassend Schritte zur Identifizierung einer bindenden Verbindung mit reduzierter Kreuzreaktivität mit einer oder mehreren Substanzen in Bezug auf die bindende Referenzverbindung unter den bindenden Kandidatenverbindungen, die Affinitäten aufweisen, die zu der der bindenden Referenzverbindung äquivalent oder größer als diese sind, wobei die weiteren Schritte Folgendes umfassen: (a) Umsetzen einer oder mehrerer Substanzen unter Bindungsbedingungen mit solchen bindenden Kandidatenverbindungen; (b) Bestimmen der Nukleotidsequenzen solcher bindenden Kandidatenverbindungen, die Komplexe mit der einen bzw. den mehreren Substanz(en) bilden; (c) Bestimmen eines Verhältnisses der Anzahl von Nukleotidsequenzen einer solchen bindenden Kandidatenverbindung, die einen Komplex mit der einen bzw. den mehreren Substanz(en) bildet, zu ihrer Gesamtzahl unter solchen bindenden Kandidatenverbindungen für jede solche bindende Kandidatenverbindung; und (d) Auswählen wenigstens einer bindenden Kandidatenverbindung aus solchen bindenden Kandidatenverbindungen, deren Verhältnis gleich dem oder kleiner als das der bindenden Referenzverbindung ist, wodurch eine nukleinsäurecodierte bindende Verbindung mit reduzierter Kreuzreaktivität für die eine bzw. die mehreren Substanz(en) in Bezug auf die bindende Referenzverbindung ohne Affinitätsverlust bereitgestellt wird.

7. Verfahren nach Anspruch 1, 2, 3, 4, 5 oder 6, wobei es sich bei den bindenden Verbindungen jeweils um einen Antikörper oder ein Antikörperfragment handelt, der bzw. das als Fusionsprotein in einem Protein-Display-System exprimiert wird.

8. Verfahren nach Anspruch 7, wobei es sich bei dem Protein-Display-System um ein Phagen-Display-System handelt.

9. Verfahren nach Anspruch 7, wobei die Probe aus der Bibliothek durch Einfangen der Antikörper oder Fragmente davon unter Verwendung eines Antikörpers, der spezifisch an eine CH1-, Kappa- oder Lambda-Kette bindet, oder unter Verwendung eines Antikörpers, der spezifisch an ein Peptid-Tag darauf bindet, erhalten wird.

10. Verfahren zur Identifizierung einer bindenden Verbindung mit erhöhter Stabilität und mit einer Affinität zu einem Liganden, die äquivalent zu der oder größer als die einer bindenden Referenzverbindung ist, wobei das Verfahren die folgenden Schritte umfasst:
Behandeln einer Bibliothek von bindenden Kandidatenverbindungen und einer bindenden Referenzverbindung mit einem a Destabilisierungsmittel, so dass eine behandelte Bibliothek bindender Verbindungen gebildet wird, wobei die bindenden Kandidatenverbindungen der Bibliothek jeweils erhalten werden, indem Aminosäuren immer an einem oder zwei Orten gleichzeitig in Bezug auf die bindende Referenzverbindung variiert werden, wobei der eine Ort bzw. die zwei Orte jeweils aus 10 bis 250 Aminosäureorten in der bindenden Referenzverbindung ausgewählt ist bzw. sind oder ein Ort aus 1 bis 500 Aminosäureorten in der bindenden Referenzverbindung ausgewählt ist, und wobei jede bindende Verbindung der behandelten Bibliothek aus einer Nukleotidsequenz besteht oder davon codiert wird;
Umsetzen eines Liganden mit der behandelten Bibliothek unter Bindungsbedingungen;
Bestimmen der Nukleotidsequenzen einer Probe von bindenden Verbindungen, die Komplexe mit dem Liganden bilden;
Bestimmen der Nukleotidsequenzen einer Probe von bindenden Verbindungen, die frei von Ligand sind, oder einer Probe von bindenden Verbindungen aus der Bibliothek;
Ordnen der Nukleotidsequenzen der bindenden Verbindungen gemäß den Affinitäten ihrer jeweiligen bindenden Verbindungen für den Liganden, wobei die Affinitäten für jede bindende Verbindung bestimmt werden, indem verglichen wird, wie viele Male eine Nukleotidsequenz damit identifiziert wird, dass die bindende Verbindung Komplexe mit dem Liganden bildet, und wie viele Male die gleiche Nukleotidsequenz damit identifiziert wird, dass die bindende Verbindung frei vom Liganden ist, oder der Anzahl der gleichen Nukleotidsequenz, die in der Probe von bindenden Verbindungen aus der Bibliothek identifiziert wird; und
Identifizieren derjenigen Nukleotidsequenzen unter der Anordnung von Nukleotidsequenzen, die bindende Verbindungen mit Affinitäten codieren, die zu der der die bindende Referenzverbindung codierenden Nukleotidsequenz äquivalent oder größer als diese sind.

11. Verfahren nach Anspruch 10, wobei es sich bei den bindenden Verbindungen um Antikörper oder Fragmente davon handelt, die von einem Protein-Display-System exprimiert werden.

12. Verfahren nach Anspruch 11, wobei es sich bei dem Destabilisierungsmittel um einen pH-Wert im Bereich von 1 bis 4 oder eine Temperatur im Bereich von 50°C bis 70°C handelt.

13. Verfahren nach Anspruch 11, wobei es sich bei dem Destabilisierungsmittel um eine Protease handelt.

14. Verfahren nach Anspruch 13, wobei die Protease aus der aus Trypsin, Chymotrypsin, Cathepsin und Endopeptidase bestehenden Gruppe ausgewählt ist.

## Revendications

1. Procédé d'identification de composés de liaison qui ont des affinités équivalentes ou améliorées pour un ligand par rapport à celle d'un composé de liaison de référence, le procédé comprenant les étapes consistant à :
faire réagir, dans des conditions de liaison, un ligand avec une banque de composés de liaison, la banque comprenant des composés-candidats de liaison et un composé de liaison de référence, dans laquelle chaque composé-candidat de liaison ainsi que le composé de liaison de référence comprennent une protéine codée par une séquence nucléotidique, et dans laquelle chacun des composés-candidats de liaison de la banque est obtenu en variant des acides aminés à un ou deux emplacements à la fois par rapport au composé de liaison de référence, dans laquelle chacun des un ou deux emplacements est choisi parmi 10 à 250 emplacements d'acides aminés dans le composé de liaison de référence ou un emplacement est choisi parmi 1 à 500 emplacements d'acides aminés dans le composé de liaison de référence ;
déterminer les séquences nucléotidiques d'un échantillon de composés de liaison formant des complexes avec le ligand ;
déterminer les séquences nucléotidiques d'un échantillon de composés de liaison étant dépourvus de ligand ou d'un échantillon de composés de liaison provenant de la banque ;
dans lequel chacun desdits échantillons contient un nombre de composés de liaison qui est au moins cinq fois la taille de la banque ;
ordonner les séquences nucléotidiques des composés de liaison en fonction des affinités de leurs composés de liaison respectifs pour le ligand, dans lequel les affinités sont déterminées pour chaque composé de liaison en comparant le nombre de fois qu'une séquence nucléotidique est identifiée avec les composés de liaison formant des complexes avec le ligand, ainsi que le nombre de fois que la même séquence nucléotidique est identifiée avec le composé de liaison dépourvu du ligand ou bien le nombre de la même séquence nucléotidique identifiée dans l'échantillon de composés de liaison provenant de la banque ; et
identifier, parmi l'ordre des séquences nucléotidiques, ces séquences nucléotidiques qui codent pour des composés-candidats de liaison ayant des affinités qui sont équivalentes à celles de la séquence nucléotidique codant pour le composé de liaison de référence ou étant supérieures à celle-ci.

2. Procédé selon la revendication 1, dans laquelle ladite étape de réaction comprend l'établissement d'une condition d'équilibre par rapport auxdits composés de liaison formant des complexes avec ledit ligand et lesdits composés de liaison dépourvus dudit ligand.

3. Procédé selon la revendication 2, dans laquelle lesdites étapes de détermination des séquences nucléotidiques desdits composés de liaison formant des complexes avec ledit ligand et de détermination de séquences nucléotidiques desdits composés de liaison dépourvus dudit un ou desdits plusieurs ligand(s) comprennent chacun un échantillonnage desdits composés de liaison, de sorte que des valeurs desdits nombres soient statistiquement significatives.

4. Procédé, selon la revendication 2, comprenant en outre des étapes destinées à l'identification d'un composé de liaison avec une solubilité accrue, par rapport au dit composé de liaison de référence, parmi lesdits composés-candidats de liaison, qui ont des affinités qui sont équivalentes à celle dudit composé de référence ou étant supérieures à celle-ci, les étapes en outre comprenant : la sélection d'au moins un composé de liaison à partir de tels composés-candidats de liaison dont l'acide nucléique codant code pour au moins un résidu d'acide aminé chargé au lieu d'un résidu d'acide aminé neutre ou hydrophobe, existant à une position équivalente dans ledit composé de liaison de référence.

5. Procédé, selon la revendication 2, comprenant en outre les étapes destinées à l'identification d'un composé de liaison avec une immunogénicité réduite, par rapport au dit composé de liaison de référence, parmi lesdits composés-candidats de liaison qui ont des affinités qui sont équivalentes à celle dudit composé de référence ou étant supérieures à celle-ci, les étapes en outre comprenant : la sélection d'au moins un composé de liaison à partir de tels composés-candidats de liaison, dont l'acide nucléique codant code pour au moins un résidu d'acide aminé différent au lieu d'un résidu d'acide aminé existant à une position équivalente dans ledit composé de liaison de référence et dont l'immunogénicité est réduite par rapport à celle dudit composé de liaison de référence.

6. Procédé, selon la revendication 2, comprenant en outre les étapes destinées à l'identification d'un composé de liaison avec une réactivité croisée réduite avec une ou plusieurs substance(s), par rapport au dit composé de liaison de référence, parmi lesdits composés-candidats de liaison qui ont des affinités qui sont équivalentes à celle dudit composé de référence ou étant supérieures à celle-ci, les étapes en outre comprenant : (a) faire réagir, dans des conditions de liaison, une ou plusieurs substance(s) avec de tels composés-candidats de liaison ; (b) déterminer les séquences nucléotidiques de tels composés-candidats de liaison formant des complexes avec l'une ou les plusieurs substance(s) ; (c) déterminer, pour chacun de ces composés-candidats de liaison, un rapport du nombre de séquences nucléotidiques d'un tel composé-candidat de liaison formant un complexe avec l'une ou les plusieurs substance(s) à son nombre total parmi de tels composés-candidats de liaison ; et (d) sélectionner au moins un composé-candidat de liaison à partir de tels composés-candidats de liaison dont le rapport est inférieur ou égal à celui du composé de liaison de référence, en fournissant de ce fait un composé de liaison codé par un acide nucléique, avec une réactivité croisée réduite pour l'une ou les plusieurs substance(s) par rapport au composé de liaison de référence, sans perte d'affinité.

7. Procédé selon les revendications 1, 2, 3, 4, 5 ou 6, dans laquelle chacun desdits composés de liaison est un anticorps ou un fragment d'anticorps exprimé en tant que protéine de fusion dans un système de présentation d'une protéine.

8. Procédé selon la revendication 7, dans laquelle ledit système de présentation d'une protéine est un système de présentation d'un phage.

9. Procédé selon la revendication 7, dans laquelle ledit échantillon provenant de ladite banque est obtenu en capturant les anticorps ou fragments de ceux-ci en utilisant un anticorps qui se lie spécifiquement à un CH1, une chaîne kappa ou lambda, ou en utilisant un anticorps qui se lie spécifiquement à une étiquette peptidique étant dessus.

10. Procédé d'identification d'un composé de liaison avec une stabilité accrue et avec une affinité pour un ligand étant équivalente à celle d'un composé de liaison de référence ou étant supérieure à celle-ci, le procédé comprenant les étapes consistant à :
traiter une banque de composés-candidats de liaison et d'un composé de liaison de référence avec un agent déstabilisant afin de former une banque traitée de composés de liaison, dans laquelle chacun des composés-candidats de liaison de la banque est obtenu en faisant varier des acides aminés à un ou plusieurs emplacement(s) à la fois par rapport au composé de liaison de référence, dans laquelle chacun des un ou deux emplacements est choisi parmi 10 à 250 emplacements d'acides aminés dans le composé de liaison de référence ou un emplacement est choisi parmi 1 à 500 emplacements d'acides aminés dans le composé de liaison de référence, et dans laquelle chaque composé de liaison de la banque traitée est constitué par une séquence nucléotidique ou est codé par celle-ci ;
faire réagir, dans des conditions de liaison, un ligand avec la banque traitée ;
déterminer les séquences nucléotidiques d'un échantillon de composés de liaison formant des complexes avec le ligand ;
déterminer les séquences nucléotidiques d'un échantillon de composés de liaison dépourvus de ligand ou d'un échantillon de composés de liaison provenant de la banque ;
ordonner les séquences nucléotidiques des composés de liaison en fonction des affinités de leurs composés de liaison respectifs pour le ligand, dans laquelle les affinités sont déterminées pour chaque composé de liaison en comparant le nombre de fois qu'une séquence nucléotidique est identifiée avec les composés de liaison formant des complexes avec le ligand, ainsi que le nombre de fois que la même séquence nucléotidique est identifiée avec le composé de liaison dépourvu du ligand ou le nombre de la même séquence nucléotidique identifiée dans l'échantillon de composés de liaison provenant de la banque ; et
identifier, parmi l'ordre des séquences nucléotidiques, ces séquences nucléotidiques qui codent pour des composés de liaison ayant des affinités qui sont équivalentes à celles de la séquence nucléotidique codant pour le composé de liaison de référence ou étant supérieures à celle-ci.

11. Procédé selon la revendication 10, dans laquelle lesdits composés de liaison sont des anticorps ou fragments d'anticorps de ceux-ci étant exprimés par un système de présentation d'une protéine.

12. Procédé selon la revendication 11, dans laquelle ledit agent déstabilisant est le pH, compris dans la gamme des 1 à 4, ou la température comprise dans la gamme des 50°C à 70°C.

13. Procédé selon la revendication 11, dans laquelle ledit agent déstabilisant est une protéase.

14. Procédé selon la revendication 13, dans laquelle ladite protéase est choisie dans le groupe constitué par la trypsine, la chymotrypsine, la cathepsine et une endopeptidase.


SEQUENCE LISTING
<110> Full Spectrum Genetics, Inc. Robert, DuBridge
<120> METHOD OF ANALYZING BINDING INTERACTIONS
<130> 900US00
<150> US 61/386,452
   <151> 2010-09-24
<150> US 61/432,529
   <151> 2011-01-13
<150> US 61/472,164
   <151> 2011-04-05
<150> US 61/510,876
   <151> 2011-07-22
<160> 5
<170> PatentIn version 3.5
<210> 1
   <211> 231
   <212> PRT
   <213> Homo sapiens
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Thr Asn Tyr
               20                  25                  30
   Gly Met Asn Trp Val Arg Gln Ala Pro Gly Lys Gly Leu Glu Trp Val
           35                  40                  45
   Gly Trp Ile Asn Thr Tyr Thr Gly Glu Pro Thr Tyr Ala Ala Asp Phe
       50                  55                  60
   Lys Arg Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Lys Tyr Pro His Tyr Tyr Gly Ser Ser His Trp Tyr Phe Asp Val
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr
   225                 230
<210> 2
   <211> 214
   <212> PRT
   <213> Homo sapiens
<400> 2
   Asp Ile Gln Met Thr Gln Ser Pro Ser Ser Leu Ser Ala Ser Val Gly
   1               5                   10                  15
   Asp Arg Val Thr Ile Thr Cys Ser Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Phe Thr Ser Ser Leu His Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Ser Thr Val Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 22
   <212> PRT
   <213> Escherichia coli
<400> 3
   Met Lys Tyr Leu Leu Pro Thr Ala Ala Ala Gly Leu Leu Leu Leu Ala
   1               5                   10                  15
   Ala Gln Pro Ala Met Ala
               20
<210> 4
   <211> 25
   <212> DNA
   <213> Escherichia coli
<400> 4
   ctagttaatt aaaggaggag caggg 25
<210> 5
   <211> 25
   <212> DNA
   <213> Escherichia coli
<400> 5
   ctaggcggcc gcaggaggag caggg 25